# EUROPEAN PATENT SPECIFICATION

(11) **EP 3 356 830 B1**
(45) Date of publication and mention of the grant of the patent: **15.06.2022**
(21) Application number: 16849945.7
(22) Date of filing: 28.09.2016
(51) Int. Cl.: G01N 33/68, C12Q 1/68

(54) **A METHOD OF TREATMENT AND PROGNOSIS**
VERFAHREN ZUR BEHANDLUNG UND PROGNOSE
MÉTHODE DE TRAITEMENT ET DE PRONOSTIC

(30) Priority: 30.09.2015 AU 2015903979
(43) Date of publication of application: 08.08.2018
(73) Proprietor: Hudson Institute of Medical Research, Clayton, VIC 3168 (AU)
(72) Inventor: EDGELL, Tracey Ann, Blind Bight, Victoria 3980 (AU); SALAMONSEN, Lois Adrienne, Kew, Victoria 3101 (AU)
(74) Representative: Gill Jennings & Every LLP
(86) International application number: PCT/AU2016/050905
(87) International publication number: WO 2017/054038

(56) References cited:
- WO-A1-2008/009705
- WO-A1-2008/009705
- WO-A1-2011/000805
- WO-A1-2014/013079
- WO-A1-2015/149129
- WO-A1-2015/149129
- A. SALMASSI: "Is granulocyte colony-stimulating factor level predictive for human IVF outcome?", HUMAN REPRODUCTION, vol. 20, no. 9, 1 January 2005 (2005-01-01), pages 2434-2440, XP055059220, ISSN: 0268-1161, DOI: 10.1093/humrep/dei071
- CHIMOTE N M ET AL: "P330 Free circulating levels of the endogenous immunoregulatory cytokine granulocyte colony stimulating factor (GCSF) correlate with implantation and clinical pregnancy in IVF cycles", HUMAN REPRODUCTION, OXFORD JOURNALS, GB, vol. 30, no. Suppl. 1, 31 May 2015 (2015-05-31), page 261, XP009509100, ISSN: 0268-1161
- Angela Morgan: "Endometrial receptivity biomarkers to predict likely IVF outcome with same cycle embryo transfer (362)", The Annual Scientific Meeting of the Endocrine Society of Australia and the Society for Reproductive Biology 2014. Poster Presentation, 25 August 2014 (2014-08-25), XP055370485, Australia Retrieved from the Internet: URL:http://esa-srb-2014.p.asnevents.com.au /days/2014-08-25/abstract/18258 [retrieved on 2017-05-09]
- LUCENA, E. et al.: "Granulocyte colony-stimulating factor (G-CSF): a mediator in endometrial receptivity for a patient with polycystic ovary (PCO) undergoing in vitro maturation (IVM)", BMJ Case Rep. 2013, 18 April 2013 (2013-04-18), XP055519978,
- CHIMOTE, N.M. et al.: "P330- Free circulating levels of the endogenous immunoregulatory cytokine granulocyte colony stimulating factor (GCSF) correlate with implantation and clinical pregnancy in IVF cycles", Meeting Info.: 31st Annual Meeting of the European -Society-of-Human-Reproduction-and-Embryol ogy (ESHRE). Human Reproduction, vol. 30, no. Suppl. 1, June 2015 (2015-06) , page 261, XP009509100,
- MORGAN, A. et al.: "Endometrial receptivity biomarkers to predict likely IVF outcome with same cycle embryo transfer (362)", The Annual Scientific Meeting of the Endocrine Society of Australia and the Society for Reproductive Biology 2014. Poster Presentation, 25 August 2014 (2014-08-25), XP055370485,
- OKULICZ, WILLIAM C.: "Analysis of serum proteins differentially expressed during simulated adequate or inadequate secretory phases in the rhesus monkey", The FASEB Journal, vol. 22, no. 2, Suppl. 113, 2008, XP055370502,
- LÉDÉE, N. et al.: "Specific and extensive endometrial deregulation is present before conception in IVF/ICSI repeated implantation failures (IF) or recurrent miscarriages", Journal of Pathology., vol. 225, no. 6, 2011, pages 554-564, XP055519984,
- RAHMATI, M. et al.: "Granulocyte-Colony Stimulating Factor Related Pathways Tested on an Endometrial Ex-Vivo Model. art e102286", PLoS ONE., vol. 9, no. 10, 2 October 2014 (2014-10-02), pages 1-6, XP055370550,
- WHITCOMB, B.W. et al.: "Circulating levels of cytokines during pregnancy; thrombopoietin is elevated in miscarriage", Fertility and Sterility., vol. 89, no. 6, June 2008 (2008-06), pages 1795-1802, XP022700012,
- OZKAN, Z.S. et al.: "What is the impact of Thl/Th2 ratio, SOCS3, IL 17, and IL 35 levels in unexplained infertility?", Journal of Reproductive Immunology., vol. 103, 2014, pages 53-58, XP028652115,
- RAHMATI, M. et al.: "P370 - Interleukin-7( IL -7),Interleukin-17 ( IL -17) and their receptors' ( IL 7-R and IL 17R) endometrial expression during implantation window, in fertile women and infertile patients", Meeting Info.: 31st Annual Meeting of the European - Society-of-Human-Reproduction-and-Embryolo gy (ESHRE). Human Reproduction, vol. 30, no. Suppl. 1, June 2015 (2015-06) , page 278, XP009509101,
- CHEN, X. et al.: "Differential expression of vascular endothelial growth factor angiogenic factors in different endometrial compartments in women who have an elevated progesterone level before oocyte retrieval, during in vitro fertilization-embryo transfer treatment", Fertility and Sterility., vol. 104, no. 4, 2015, pages 1030-1036, XP029285527,
- LIANG, P.Y. et al.: "The proinflammatory and anti-inflammatory cytokine profile in peripheral blood of women with recurrent implantation failure", Reproductive BioMedicine Online ., vol. 31, no. 6, 21 August 2015 (2015-08-21), pages 823-826, XP029328885,
- SALAMONSEN, L.A. et al.: "The Microenvironment of Human Implantation: Determinant of Reproductive Success", American Journal of Reproductive Immunology., vol. 75, 2016, pages 218-225, XP055519989,
- BINDER, N.K. et al.: "Placental Growth Factor Is Secreted by the Human Endometrium and Has Potential Important Functions during Embryo Development and Implantation, art e0163096", PLoS One., vol. 11, no. 10, 2016, pages 1-15, XP055370634,
- ZHAO, J. et al.: "Whether G-CSF administration has beneficial effect on the outcome after assisted reproductive technology? A systematic review and meta-analysis", Reproductive Biology and Endocrinology., vol. 14, no. 1, 2016, XP055370635,
- EDGELL, T.A. et al.: "Assessing receptivity in the endometrium: the need for a rapid, non-invasive test", Reproductive BioMedicine, vol. 27, no. 5, 2013, pages 486-496, XP028762754,
- Suneeta Senapati ET AL: "Biomarkers for ectopic pregnancy and pregnancy of unknown location", Fertility and Sterility, vol. 99, no. 4, 1 March 2013 (2013-03-01), pages 1107-1116, XP055148369, ISSN: 0015-0282, DOI: 10.1016/j.fertnstert.2012.11.038
- IMAI-SENGA Y ET AL: "A human gene, ATP6E1, encoding a testis-specific isoform of H^+-ATPase subunit E", GENE, ELSEVIER AMSTERDAM, NL, vol. 289, no. 1-2, 1 May 2002 (2002-05-01) , pages 7-12, XP004358916, ISSN: 0378-1119, DOI: 10.1016/S0378-1119(02)00542-5
- NATALIE K. BINDER ET AL: "Placental Growth Factor Is Secreted by the Human Endometrium and Has Potential Important Functions during Embryo Development and Implantation, art e163096", PLOS ONE, vol. 11, no. 10, 6 October 2016 (2016-10-06), pages 1-15, XP055370634, DOI: 10.1371/journal.pone.0163096
- EDGELL TRACEY A ET AL: "Assessment of potential biomarkers of pre-receptive and receptive endometrium in uterine fluid and a functional evaluation of the potential role of CSF3 in fertility", CYTOKINE, ACADEMIC PRESS LTD, PHILADELPHIA, PA, US, vol. 111, 5 September 2018 (2018-09-05), pages 222-229, XP085557965, ISSN: 1043-4666, DOI: 10.1016/J.CYTO.2018.08.026

## Description

### FILING DATA

This application is associated with and claims priority from Australian Provisional Patent Application No. 2015903979, filed on 30 September 2015, entitled "A method of treatment and prognosis".

### BACKGROUND

### FIELD

The present disclosure teaches an assay to determine the likelihood of a successful implantation of an embryo into a female subject leading to a pregnancy and a method of treatment to facilitate same. Enabled herein is an improved assisted reproduction technology protocol and the identification of therapeutic targets. Taught herein is a composition comprising reagents required for prognostic evaluation and treatment.

### DESCRIPTION OF RELATED ART

Bibliographic details of the publications referred to by author in this specification are collected alphabetically at the end of the description.

Reference to any prior art in this specification is not, and should not be taken as, an acknowledgment or any form of suggestion that this prior art forms part of the common general knowledge in any country.

Assisted reproductive technology (ART) has had a major impact on the ability for human females to achieve pregnancy with a successful outcome.

However, the ART process can be an emotional and stressful experience on recipients. The ability to reduce unsuccessful implantation outcomes would assist in reducing this stress and reducing overall costs of the procedure.

As an indicator of the prevalence of ART in the human clinical setting, a report by Macaldowie et al. (2013) Assisted reproductive technology in Australia and New Zealand 2011, Sydney: National Perinatal Epidemiology and Statistics Unit, the University of New South Wales, Australia, indicates that there was an 8.3% increase in Australian women undertaking this procedure in 2011. In 95.1% of cases, women used their own (autologous) oocytes or embryos. Approximately one third of women used frozen/thawed embryos. Despite this increase in the use of assisted reproductive technology, rates of successful clinical pregnancies are not overly high. In fact, in 2011, only 23.1% resulted in clinical pregnancy and 17.5% in live births (Macaldowie *et al.* (2013) *supra).*

A range of parameters, such as, age of the recipient, autologous and oocyte/embryo recipient cycles and use of fresh compared to frozen/thawed embryos has been investigated on their impact on the success or otherwise of ART. However, there is still a need for a knowledge-based assessment protocol to assist a clinician to determine whether the uterus of a female recipient is optimally prepared for embryo receptivity and to identify therapeutic targets to facilitate successful ART.

WO2008/009705 relates generally to an assay for determining the implantation potential of a plurality of embryos, each obtained or to be obtained by assisted fertilisation of an oocyte of a female subject, the assay comprising measuring levels of G-CSF in the follicular fluid present in the follicle from which the oocyte is derived, and determining the implantation potential of each embryo from the level of measured follicular fluid G-CSF.

WO2015/149129 describes an assay for determining the likelihood of a successful implantation of an embryo into a female subject leading to pregnancy, the assay comprising determining the level of biomarkers IL-8, G-CSF and/or VEGFA in a biological fluid sample taken before embryo implantation.

WO2014/013079 relates generally to methods for determining endometrial/uterine receptivity by measuring biomarkers of the activity state of uterine natural killer cells, such as IL-6 and IL-12.

Chimote (2015) is an Abstract published in the proceedings of the 31st annual meeting of the European Society of Human Reproduction and Embryology (ESHRE) and merely reports on the difference in serum G-CSF levels between pregnant and non-pregnant women following blastocyst transfer.

Morgan A. (2014) discloses the CSF3 is elevated and CSFR3 is diminished in women undergoing *in vitro* fertilisation for whom pregnancy was unsuccessful.

WO2011/000805A1 discloses a method for determining the implantation potential of an oocyte from a female subject.

### SUMMARY

The invention is defined in the claims. The use of embryos and methods of assisted reproduction are not part of the invention. The present description teaches an improved protocol for assisted reproduction technology. In an embodiment, at the 2 day post ovulation induction trigger (OI+2 days) such as human chorionic gonadotropin trigger (hCG+2 days), gonadotropin releasing hormone analog trigger (GnRHa+2 days) or another drug trigger plus 2 days or early secretory phase equivalent, a biological fluid sample is taken to assess the receptivity of the endometrium. Whilst the "+ 2 days" provides a useful time period, the described protocol encompasses a time period of at least from 1 day to 5 days. An early stage equivalent includes a natural or stimulated cycle. A natural cycle, for example, would be luteinizing hormone (LH) + 2 days (LH + 2 days). Again, the time period may range from 1 day to 5 days. This described protocol, which is not part of the invention, envisages the use of a frozen embryo in an implantation protocol. The biological sample includes a uterine sample or a blood, plasma, serum, ascites, lymph fluid, tissue exudate or urine sample. The level or glycosylated form of CSF3 or its receptor determine the degree of likelihood of a successful implantation of a fertilized embryo in that cycle and further whether a pregnancy is likely to be a clinical or preclinical pregnancy. Furthermore, antagonizing CSF3 activity including CSF3-mediated signaling and/or agonizing CSF3 receptor is proposed to improve the likelihood of a successful pregnancy. The improved protocol enables a clinician to decide whether or not to proceed with embryo transfer or to freeze and store the embryo for subsequent use or to treat the female patient to enhance pregnancy success. Notwithstanding, the protocol can also be used to test endometrial receptivity in an assisted reproductive technology protocol. A composition is encompassed herein comprising reagents required to perform the protocol and to facilitate treatment.

The present specification teaches, therefore, an endometrial receptivity test performed prior to the time window for implantation (i.e. at OI +5 days, e.g. hCG +5 days or GnRHa+5 days or mid-secretory phase equivalent). The assay developed in accordance with the present invention is able to be completed and the results interpreted in a timely manner to minimize interference in the uterine cavity at the time of embryo transfer/implantation. This reduces stress on a potential recipient, is efficient and has a high level of predictive outcome. Notwithstanding, the subject assay is also applicable to screening recipients for implantation of frozen embryos. The assay is knowledge-based in the context of a comparison of levels of biomarkers or ratios of levels of 2 or more biomarkers relative to control levels or ratios. Hence, provided herein is a knowledged-based assessment protocol which enables a clinician to determine the relative likelihood that an embryo transfer will lead to a successful clinical pregnancy. Further enabled herein is a method for treating a female patient to improve the likelihood of a successful pregnancy.

The assay herein enables determination of predicted receptivity in a female subject during either a stimulated or natural cycle to ascertain whether a viable receptive endometrium will be present at the time of embryo transfer. This aids the clinician in determining whether to proceed with embryo transfer or to freeze embryos and wait for a natural cycle or alternatively another treated cycle in which uterine conditions are more favorable for a successful embryo transfer and implantation. The protocol can also be used to assess endometrial receptivity prior to hormonal stimulation.

In an embodiment, the sample is a uterine sample in the form of a uterine lavage sample obtained at the time of egg collection (two days after ovulation induction by hCG [hCG+2 days], GnRHa (GnRHa+2 days) or another drug (OI+2 days) in hormone stimulated cycles (or early secretory phase equivalent). Alternatively, the sample is blood, plasma or serum, ascites, lymph fluid, tissue exudate or urine. The biomarker examined is CSF3. The assay determines favorable endometrial receptivity when CSF3 is low. A comparison of levels can be made to those who do become pregnant *versus* those who do not become pregnant. Hence, CSF3 is a biomarker of female utility. Without limiting to any one theory or mode of action, it is proposed that CSF3 and its receptor influence embryo attachment within the uterine environment and influences endometrial development.

Importantly, the previous practice of the clinical use of CSF3 as an adjuvant to improve implantation rates appears misguided and contraindicated. It is proposed herein to neutralize excess CSF3 in order to improve pregnancy success.

Hence, enabled herein is an assay for stratifying a female subject with respect to likely outcome of embryo implantation, the outcome selected from pregnancy and no pregnancy, the assay comprising, determining the concentrations of CSF3 or CSF3 receptor from a fluid sample from the subject, wherein an elevated level of CSF3 or reduced level of CSF3 receptor relative to a control provides an indication of a poor likelihood of a successful pregnancy. Normalized CSF3 and CSF3 receptor levels are indicative of a likelihood of a successful pregnancy.

Further enabled herein is an assay to stratify a female subject undergoing an assisted reproductive technology protocol with respect to the likelihood of pregnancy or no pregnancy and if pregnant, whether the pregnancy is a clinical or preclinical pregnancy, the assay comprising determining the levels of CSF3 and/or CSF3 receptor in a fluid sample from the subject, wherein a successful pregnancy is considered likely when levels of CSF3 are normal relative to a control or CSF3 receptor is high; a successful pregnancy is considered less likely when levels of CSF3 are high or CSF receptor is low relative to a control. A multivariate analysis may include other biomarkers (e.g. VEGF, IL-6, IL-8, IL-17A, CRP, P1GF, sFlt-1 and/or sGP130) from more than one source (e.g. uterine lavage and serum) or biomarkers with body mass index (BMI) and age. As taught herein, an elevation in any one or more of VEGF, IL-6, IL-8, IL-17A, CRP, PIGF sFlt-1 and/or sGP130 relative to a control is supportive of an unlikely successful pregnancy outcome. It is proposed that any one or more of these markers would be assayed together with CSF3 or CSF3 receptor. In addition, or as an alternative, individual, stand alone assays can be conducted using CSF3, IL-17A and/or PlGF. Other markers such as progesterone may also be measured along with BMI and age. In addition, lower than normalized levels of CSF3 are correlated to a higher incidence of miscarriage.

Taught herein is a kit to undertake an assay to determine levels of CSF3 or its receptor. A computer program is also contemplated herein to assist in the analysis of data. In an embodiment, the assay enabled herein may be used in existing knowledge-based architecture or platforms associated with pathology services. For example, results of the assays are transmitted *via* communications network (e.g. the internet) to a processing system in which an algorithm is stored and used to generate a predicted posterior probability value which translates to an index of likelihood of endometrial receptivity or non-receptivity leading to a successful pregnancy which is then forwarded to the clinician in the form of a prognostic or predictive report.

Taught herein, therefore, is a composition or kit or computer-based system which comprises reagents necessary to detect the concentration of CSF3 or its receptor and the computer hardware and/or software to facilitate determination and transmission of reports to the clinician. Reference to "levels" of the biomarkers also encompasses determination of ratios CSF3 to its receptor. The kit may also comprise reagents to detect levels of one or more of VEGF, IL-6, IL-8, IL-17A, CRP, PlGF, sFlt-1 and/or sGP130. In an embodiment, the kit enables detection of at least one of CSF3, IL-17A and/or PlGF.

The concepts developed herein could assist in the development of medicaments to facilitate endometrial receptivity and to assist in a more favorable outcome of clinical pregnancy. Such medicaments include CSF3 antagonists and CSF3 receptor agonists useful therapeutic agents or adjuvants to improve pregnancy success.

A list of abbreviations is provided in Table 1.

**Table 1**

| ***Abbreviations*** | |
|---|---|
| **Abbreviation** | **Definition** |
| +2 | + 2 days |
| +5 | + 5 days |
| ART | Assisted reproduction technology |
| AUC | Area under the curve |
| BMI | Body mass index |
| CRP | C-reactive protein |
| CSF3 | Colony stimulating factor 3 (G-CSF) |
| CSF3R | Colony stimulating factor receptor |
| G-CSF | Granulocyte-colony stimulating factor also known as CSF3 or colony stimulating factor 3 |
| GnRHa | Gonadotropin releasing hormone analog |
| GnRHa+2 | Early secretory phase equivalent in a cycle in which GnRHa (or another stimulus) is used to trigger ovulation |
| GnRHa+5 | Mid secretory phase equivalent in a cycle in which GnRHa (or another stimulus) is used to trigger ovulation |
| hCG | Human chorionic gonadotropin |
| hCG+2 | Early secretory phase equivalent in a cycle in which hCG (or another stimulus) is used to trigger ovulation |
| hCG+5 | Mid secretory phase equivalent in a cycle in which hCG (or another stimulus) is used to trigger ovulation |
| IL-6 | Interleukin-6 |
| IL-8 | Interleukin-8 |
| IL-17A | Interleukin-17A |
| IVF | *In vitro* fertilization |
| LH | Luteinizing hormone |
| OI | Ovulation induction |
| 01+2 | Early secretory phase equivalent in a cycle in which a drug is used to stimulate ovulation |
| 01+5 | Mid secretory phase equivalent in a cycle in which a drug is used to stimulate ovulation |
| ROC | Receiver operating characteristics |
| sFlt-1 | Soluble fms-like tyrosine kinase |
| sGP130 | Soluble glycoprotein 130 |
| VEGFA | Vascular endothelial growth factor |

### BRIEF DESCRIPTION OF THE FIGURES

**Figure 1** is a graphical representation showing the concentrations of biomarkers present in uterine lavage collected during the early secretory phase of natural cycle from fertile and idiopathic infertile women.

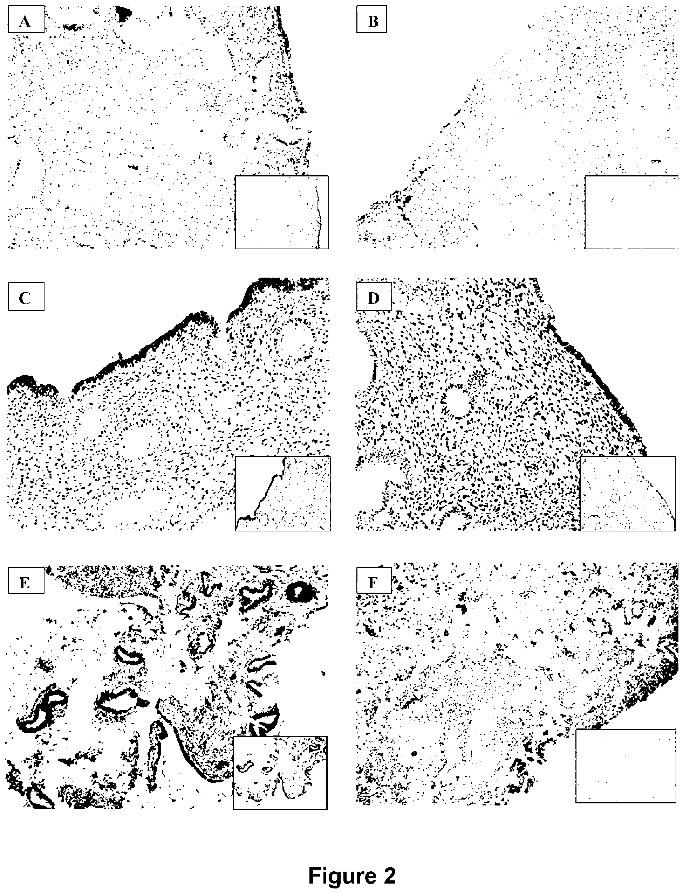

**Figure 2** is a photographical representation showing immunohistochemistry of CSF3 and CSF3 receptor in the endometrium of naturally cycling fertile and infertile women during the early secretory phase, and women undergoing IVF stimulation at hCG+2. Shown are representative images of CSF3 staining in fertile (A) and infertile (B) and CSF3 receptor staining in fertile (C) and infertile (D) endometrium. Additional images from tissues collected at hCG+2 in IVF stimulation cycles, of infertile women who became pregnant (E) or did not become pregnant (F) are also shown. Negative control IgG staining is shown.
**Figure 3** is a graphical representation showing scoring of IHC in cellular compartments glandular epithelium (GE) luminal epithelium (LE) and stroma (S) of early secretory phase endometrium collected from fertile (F) and primary idiopathic infertile (PIF) women.
**Figure 4** is a graphical representation showing scoring of IHC in glandular epithelium (GE) of endometrium collected from women undergoing ART, classified as having outcome of pregnancy or no pregnancy, alongside fertile egg donors.
**Figure 5** is a graphical representation showing the effect of adhesion of ECC1 cells by treatment with glycosylated and non-glycosylated CSF3 at high and low concentration. Effect of acute exposure and chronic (24 hour pre-exposure).
**Figure 6** is a graphical representation showing the effect on proliferation of ECC1 cells by treatment with non-glycosylated (A) and glycosylated CSF3 (B) at high (a) and low (b) concentration. Effect of acute exposure and chronic (24 hour pre-exposure). ^{∗}p<0.05, ^{∗∗}p<0.01, ^{∗∗∗}p<0.001, ^{∗∗∗∗}p<0.0001.
**Figure 7** is a graphical representation showing effect of non-glycosylated (a) and glycosylated (b) CSF3 on trophoblast cell migration .
**Figure 8** is a graphical representation showing the effect of non-glycosylated (a) and glycosylated (b) CSF3 on trophoblast cell invasion.
**Figure 9** is a schematic representation of a serum cohort stratified in Examples 7. Final patient outcomes were determined with respect to live births.
**Figure 10** is a graphical representation of the predictive index of serum assay collected from 106 women undergoing a same cycle embryo transfer. Serum was collected at hCG+2 at the time of oocyte collection. Outcomes were ascribed as pregnancy (fetal heartbeat) or no pregnancy (inclusive of biochemical/pre-clinical pregnancy).
**Figure 11** is a graphical representation of the predictive index of serum assay collected from 106 women undergoing a same cycle embryo transfer. Serum was collected at hCG+2 at the time of oocyte collection. Outcomes were ascribed as pregnancy (fetal heartbeat) or no pregnancy (inclusive of biochemical/re-clinical pregnancy). ANOVA with Tukey post hoc statistical analysis performed to compare PI values between groups, ^{∗∗∗∗}p<0.0001.

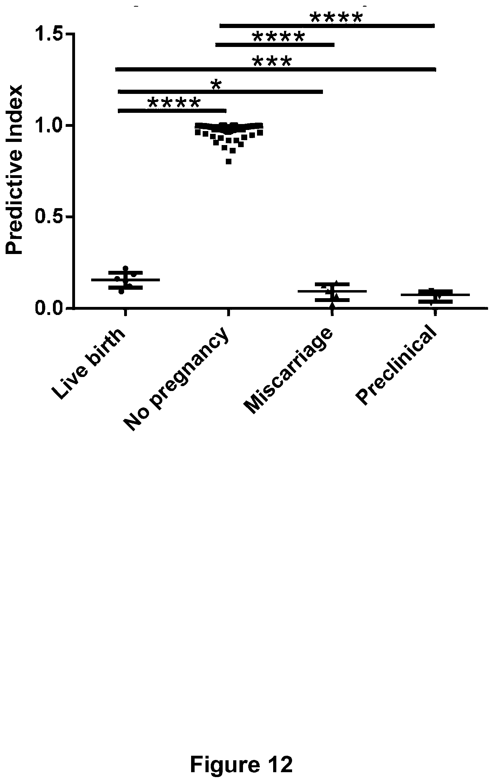

**Figure 12** is a graphical representation of the predictive index of serum assay collected from 106 women undergoing a same cycle embryo transfer and modeled in a 4 way outcome model. Serum was collected at hCG+_2 at the time of oocyte collection. Outcomes were ascribed as live birth, miscarriage, no pregnancy and preclinical/biochemical pregnancy in a 4 way logistic regression model. ANOVA with Tukey post hoc statistical analysis performed to compare PI values between groups, ^{∗∗∗∗}p<000.1, ^{∗∗∗}p<0.001, ^{∗}p<0.01.
**Figure 13** is a graphical representation of the predictive index of serum assay collected from 174 women undergoing a same cycle embryo transfer. Serum was collected at4 hCG+2 at the time of oocyte collection. Outcomes were ascribed as pregnancy (fetal heartbeat) or no pregnancy (inclusive of biochemical/pre-clinical pregnancy) and a 2 way model generated. T-test ^{∗∗∗∗}p<0.0001.

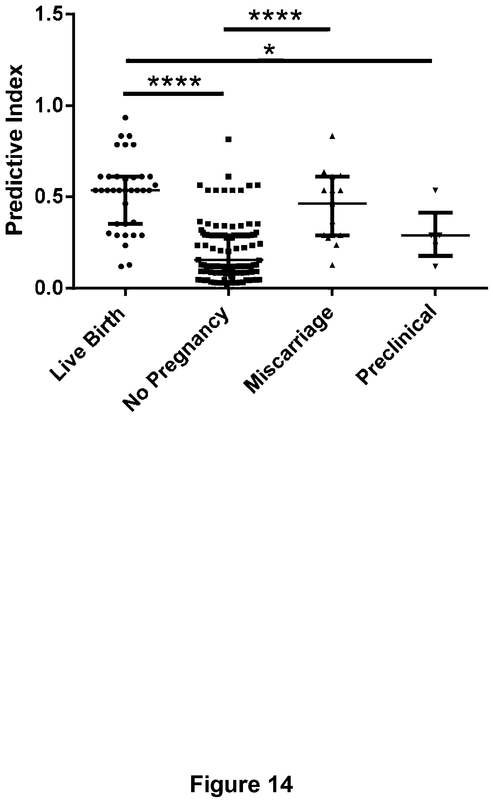

**Figure 14** is a graphical representation of the predictive index of serum assay collected from 173 women undergoing a same cycle embryo transfer. Serum was collected at hCG+2 at the time of oocyte collection. Outcomes were ascribed as live birth, no pregnancy, miscarriage or biochemical/pre-clinical pregnancy) and PI values arising from 2 way model compared. ANOVA with Tukey post hoc statistical analysis performed to compare PI values between groups, ^{∗∗∗∗}p<0.0001.

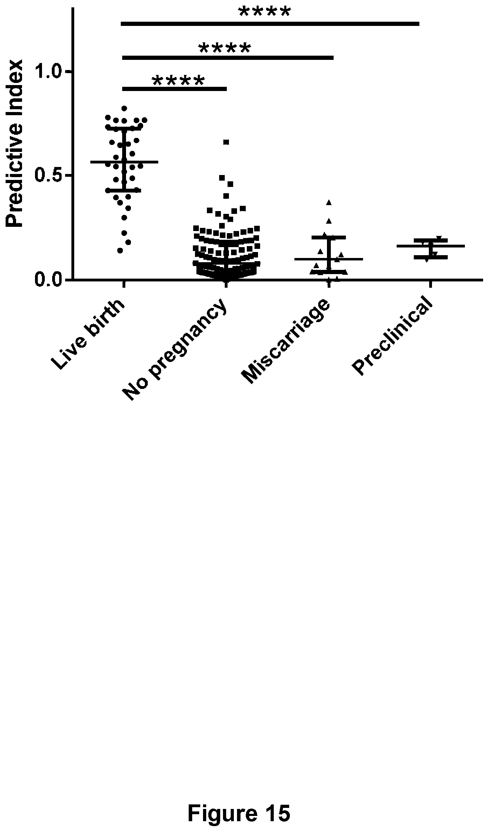

**Figure 15** is a graphical representation of the predictive index of serum assay collected from 173 women undergoing a same cycle embryo transfer and modeled in a 4 way outcome model. Serum was collected at hCG+2 at the time of oocyte collection. Outcomes were ascribed as live birth, no pregnancy, miscarriage or biochemical/pre-clinical pregnancy) and PI values arising from 4 way logitboost model. ANOVA with Tukey post hoc statistical analysis performed to compare PI values between groups, ^{∗∗∗∗}p<0.0001, ^{∗∗∗}p<0.001, ^{∗}p<0.01.
**Figures 16I through III** are graphical representations of IL-17A levels and pregnancy outcomes.
**Figure 17** is a graphical representation showing that lower than normalized levels of CSF3 is indicative of a higher incidence of miscarriage.

### DETAILED DESCRIPTION

Throughout this specification, unless the context requires otherwise, the word "comprise" or variations such as "comprises" or "comprising", will be understood to imply the inclusion of a stated element or integer or method step or group of elements or integers or method steps but not the exclusion of any element or integer or method step or group of elements or integers or method steps.

As used in the subject specification, the singular forms "a", "an" and "the" include plural aspects unless the context clearly dictates otherwise. Thus, for example, reference to "a biomarker" includes a single biomarker, as well as two or more biomarkers; reference to "an embryo" includes a single embryo, as well as two or more embryos; reference to "the disclosure" includes single and multiple aspects taught by the disclosure; and so forth. Reference to a "sample" includes a uterine sample, or a sample of blood, plasma or serum, ascites, lymph fluid, tissue exudate or urine fluid.

The use of numerical values in the various ranges specified in this specification, unless expressly indicated otherwise, are stated as approximations as though the minimum and maximum values within the states ranges were both preceded by the word "about". Furthermore, the manner of collection, the volume of fluid and level of concentration (e.g. in lavage fluid *versus* aspirate) will result in different ranges. Notwithstanding, the skilled person would compensate for the "low" and "high" values given here for a particular range, without departing from the scope of the present invention. Also, the disclosure of these ranges is intended as a continuous range including every value between the minimum and maximum values. In addition, the subject protocol extends to ratios of two or more markers providing a numerical value associated with a level of likelihood of a successful embryo transfer leading to a clinical pregnancy. A dilution factor for lavage is determined using the estimation: dilution factor = serum concentration divided by lavage urea concentration. Reference to "OI+2" means two days post ovulation induction by human chorionic gonadotropin (hCG) or by another drug or hormone such as but not limited to gonadotropin releasing hormone analog (GnRHa). A "control" generally means the level of a biomarker in a healthy individual either prior to or following pregnancy. Conveniently, a control is a statistically validated level.

Reference to "success" in relation to a pregnancy does not necessary imply that the pregnancy will go to term. Reference to a successful pregnancy means progression to a clinical pregnancy regardless of the ultimate result of the pregnancy. The aim is to distinguish between pregnancy, preclinical pregnancy and no pregnancy. The subject method can also distinguish the likelihood of a live birth from miscarriage based on levels of CSF3 lower than normalized levels.

A rapid, efficient and sensitive assay of endometrial receptivity is provided for the identification of human female subjects in whom an outcome of a successful pregnancy is likely following assisted reproductive technology. This enables the development of an improved assisted reproduction technology protocol by the normalization or antagonism of excess CSF3 and/or enhancing CSF3 receptor (CSFR) levels.

The present disclosure teaches an assay to stratify potential human female recipients for autologous, frozen/thawed or heterologous embryo implantation in terms of likelihood or otherwise for a successful pregnancy based on the level of CSF3 or CSF3-mediated signaling and/or CSF3R. As indicated above, a successful pregnancy means progression to a clinical pregnancy. In essence, CSF3 and/or CSF3R levels are determined from a fluid sample which discriminate patients on likelihood of a pregnancy or no pregnancy and preclinical pregnancy or clinical pregnancy in the cycle of sampling and transfer. These biomarkers are also useful for determining the success or otherwise of a recipient achieving pregnancy using a donated egg or a donated fertilized embryo. The biomarkers may be considered alone or in combination with patient physical characteristics such as BMI and age. Reference to a "fluid sample" includes uterine lavage, blood, plasma and serum, ascites, lymph fluid, tissue exudate and urine. CSF3 and CSF3R may be determined in a fluid sample from one source or from two or more sources (e.g. uterine lavage and serum).

The biomarkers comprise CSF3 and CSF3R as well as indicators of CSF3-mediated signaling. The levels of CSF3, CSF3R and/or indicators of CSF3-mediated signaling, including ratios of levels of these biomarkers, are determined relative to a control. A control includes levels in a cohort of subjects under study or may be a statistically determined knowledge database obtained over a large number of studies. Reference is made to a "first knowledge base" which comprises data in the form of correlations between levels or ratios of levels of biomarkers and certain pregnancy outcomes. A second knowledge base represents data from a recipient undergoing the prognostic assay. Data in the second knowledge base are compared to the first knowledge base for a determination of the likelihood or otherwise of a successful pregnancy. In addition, biomarkers may be subject to multivariate analysis with physical parameters such as BMI and age as well as from two different sources.

In essence, a human female subject may present for egg collection following a natural cycle or after hormone stimulated therapy. Alternatively, the protocol is independent of egg collection. The levels or ratios of CSF and/or CSF3R measured at this time are useful for determining whether to proceed following fertilization of an embryo or to wait for another cycle. In an embodiment, an elevated level of IL-8 of CSF or a low level of CSF3R during this cycling phase are indicators of a poor likelihood of a successful pregnancy. Alternatively, normal (low) levels of CSF3 or normal (high) levels of CSF3R each relative to a control are indicators for a greater likelihood of a successful pregnancy. The assay results also apply to endometrial receptivity prior to hormonal stimulation in an assisted reproduction technology protocol and such an assay is contemplated herein.

In an embodiment, at the time of egg collection the female subject is generally sedated and this is a convenient time to obtain a uterine sample to test levels of the biomarkers. If the intent is to use a frozen embryo a female subject may in any event be sedated. In an embodiment, a soft catheter is used to infuse 1-10ml of saline which is then recovered as uterine lavage. Whilst an aspirate sample is also suitable, for the purposes of the present protocol, a uterine lavage is considered optimal. Reference to "1-10ml" of saline includes 1, 2, 3, 4, 5, 6, 7, 8, 9 and 10ml. A range of 1-3ml saline is considered optimal. The sample may be tested for protein levels or levels of corresponding mRNA which encodes for CSF3 or CSF3R. Aspirates may be in the 5-1,000µl range. In another embodiment, the level of CSF3 or CSF3R are determined from blood, plasma or serum, ascites, lymph fluid, tissue exudate or urine. In a further embodiment, CSF3 and/or CSF3R are determined alone or in combination with BMI and age.

Conveniently, at the time of sedation of the female subject and collection of the uterine sample, the levels and/or ratios of CSF3 and/or CSF3R are determined to stratify the subject on expected outcome of embryo implantation. Outcomes include pregnancy or no pregnancy. For those subjects who achieve pregnancy, the biomarkers can also predict a clinical or preclinical pregnancy. In an embodiment, normal levels of CSF3 relative to a control is indicative of a likely outcome of pregnancy. Elevated CSF3 levels are indicative of a reduced likelihood of pregnancy. A reduced CSF3R level on the other hand is more likely to be associated with a poor pregnancy outcome. As indicated above, the assay can be used to assay endometrial receptivity prior to stimulation in an assisted reproduction technology protocol.

For those subjects achieving pregnancy, elevated normal level of CSF3R relative to a control is indicative of a likelihood of a clinical pregnancy compared to a preclinical pregnancy which is associated with reduced levels of CSF3R.

Hence, the present disclosure teaches a method for stratifying a human female subject with respect to expected outcomes of embryo implantation in an assisted reproduction technology protocol, the method comprising determining the levels of CSF3 and/or CSF3R in a fluid sample, wherein elevated levels of CSF3 relative to a control are indicative of a low likelihood of a successful pregnancy and reduced levels of CSF3R relative to a control are indicative of a low likelihood of a successful pregnancy and where normal CSF3 levels relative to a control is indicative of high successful pregnancy outcome and normal levels of CSF3R relative to a control are indicative of high successful pregnancy outcome.

Hence, elevated CSF3 levels are suggestive of a low likelihood of a successful pregnancy. Additional markers or factors may also be determined including levels of VEGF, IL-6, IL-8, IL-17A, CRP, PlGF, sFlt-1 and/or sGP130. Progesterone, level of BMI and the patient's age are also useful parameters to measure. Elevated levels of these biomarkers are indicators of poor likelihood of a successful pregnancy.

The present disclosure teaches a method for stratifying a human female subject with respect to expected outcomes of embryo implantation in an assisted reproduction technology protocol, the method comprising determining the levels of IL-17A in a fluid sample, wherein elevated levels of IL-17A relative to a control are indicative of a low likelihood of a successful pregnancy and where normal IL-17A levels is indicative of high likelihood of a successful pregnancy outcome.

Also taught herein is a method for stratifying a human female subject with respect to expected outcomes of embryo implantation in an assisted reproduction technology protocol, the method comprising determining the levels of PIGF in a fluid sample, wherein elevated levels of PIGF relative to a control are indicative of a low likelihood of a successful pregnancy and where normal PIGF levels is indicative of high likelihood of a successful pregnancy outcome.

Still further taught herein is a method for stratifying a human female subject with respect to expected outcomes of embryo implantation in an assisted reproduction technology protocol, the method comprising determining the levels of CSF3 and/or CSF3R in a fluid sample together with IL-17A and/or PlGF, wherein elevated levels of CSF3 relative to a control are indicative of a low likelihood of a successful pregnancy and reduced levels of CSF3R relative to a control are indicative of a low likelihood of a successful pregnancy and elevated levels of IL-17A and/or PIGF relative to controls are indicative of low likelihood of successful pregnancy and where normal CSF3 levels is indicative of high likelihood of a successful pregnancy outcome and normal levels of CSF3R are indicative of high likelihood of a successful pregnancy outcome and normal levels of IL-17A and/or PIGF are indicative of high likelihood of a successful pregnancy outcome, all relative to controls.

Contemplated herein is an assay for stratifying a female subject with respect to likely outcome of embryo implantation, the outcome selected from pregnancy and no pregnancy, the assay comprising, determining the concentrations of CSF3 and/or CSF3R from a uterine sample from the subject wherein the level of CSF3 or CSF3R or ratio of levels of CSF3 and CSF3R provides an indication of the likelihood of a successful pregnancy.

Enabled herein is a multiplex assay to stratify a female subject undergoing an assisted reproductive technology protocol with respect to the likelihood of pregnancy or no pregnancy, the assay comprising determining the levels of CSF3 and/or CSF3R in a fluid from the subject, wherein a successful pregnancy is considered likely when levels of CSF3 are normal; a successful pregnancy is considered less likely when levels of CSF3 are high; a successful pregnancy is considered more likely if levels of CSF3R are normal; a successful pregnancy is considered less likely if levels of CSF3R are low. These levels are relative to a control.

In an embodiment, the fluid sample is a uterine lavage sample. In another embodiment, the fluid sample is blood, plasma, serum, ascites, lymph fluid, tissue exudate or urine.

In an embodiment, the female recipient is either undergoing hormone assisted cycle or is naturally cycling (e.g. with luteinizing hormone). In an embodiment, endometrial receptivity is assessed prior to initiation of any hormonal assisted cycling.

Whilst it is most convenient for the uterine sample to be taken once during egg collection, the present protocol does not preclude multiple samples being obtained. However, optimally, the female subject is sampled once at the time of egg collection or, for female subjects having a frozen/thawed embryo, the sample is conducted at OI+2 (e.g. hCG+2 or GnRH+2 or OI trigger by another drug) days for hormonal assisted cycles or at an early secretory phase equivalent. Ovulation induction may be by hCG or another drug such as but not limited to GnRHa. Blood or other fluid sample may be taken at any time and is far less invasive. Conveniently, non-lavage samples are taken at the same time that the patient presents for egg collection.

The levels or ratios of levels of CSF3 and/or CSF3R may be compared to the same subject as she undergoes cycling or is in a non-cycling phase or may be compared to a cohort of females undergoing a similar procedure or may be compared to a first knowledge database collected over a statistical number of trials. As indicated above, the levels of the biomarkers determined in a test subject represent a second knowledge base of unknown predictive outcomes. The second knowledge base is compared to the first knowledge base.

Whilst not intending to limit the interpretation of the subject method to particular values of "elevated" or "reduced" (or normal) levels of biomarkers, an example of reduced *versus* elevated CSF is 0-2,000 picograms/ml sample for reduced and 3,000 to 10,000 picograms/ml sample for elevated. These numbers are representative from a 3ml uterine lavage and will alter depending on the precise method of sample collection.

Those are based on a Bioplex 200 instrument (Biorad Laboratories, Berkely, CA, USA) platform. Ranges may differ based on the pathology platform used.

The present invention is not to be limited to these ranges across all female subjects but the ranges given here provide a guide only as to likely levels which will be monitored by a clinician. In addition, physical characteristics of the patient may be considered such as BMI and age.

In particular, the stratification assay enabled herein may be used in existing knowledge-based architecture or platforms associated with pathology/clinical services. In an embodiment, the results from the assays are transmitted *via* a communications network (e.g. the internet) to a processing system in which an algorithm is stored and used to generate a predicted posterior probability value which translates to a probability of a particular outcome which is then forwarded to an end user (e.g. clinician) in the form of a prognostic or predictive report.

The assay may, therefore, be in the form of a kit or computer-based system which comprises the reagents necessary to detect the concentration of the biomarkers and the computer hardware and/or software facilitates determination and transmission of reports to a clinician.

Enabled herein is a prognostic assay for stratifying a female subject with respect to likely outcomes of embryo implantation, the outcome selected from pregnancy and no pregnancy, the assay comprising determining the concentration of CSF3 and/or CSF3R in a fluid sample from the subject; wherein the level of CSF3 or CSF3R or ratio of levels of CSF3 and CSF3R provides an indication of the likelihood of a successful pregnancy. Measuring both CSF3 and CSF3R is likely to increase the level of sensitivity and specificity of the prognostic assay result. Additional biomarkers may also be included without departing from the scope of the subject invention.

Further enabled herein is a prognostic assay for stratifying a female subject with respect to likely outcomes of embryo implantation, the outcome selected from pregnancy and no pregnancy, the assay comprising determining the concentration of CSF3 and/or CSF3R in a fluid sample from the subject. In addition, the "outcome" includes a miscarriage or a likelihood that the pregnancy will not go to term.

In an embodiment, the fluid sample is uterine lavage. In an embodiment, the fluid sample is blood, plasma, serum, ascites, lymph fluid, tissue exudate or urine. In an embodiment, the biomarkers are determined in two or more different fluid samples.

In an embodiment, taught herein is an assay for stratifying a female subject with respect to likely outcome of embryo implantation, the assay comprising determining the concentration of CSF3 and/or CSF3R in a fluid sample from the subject; subjecting the levels to an algorithm generated from a first knowledge base of data comprising the levels of the same biomarkers from subjects of known status with respect to the outcome wherein the algorithm provides an index of probability of the subject proceeding or not proceeding with a pregnancy. Reference to the "algorithm" is an algorithm which performs a multivariate analysis function. The latter may include factors such as BMI and age. The algorithm may further distinguish between clinical and preclinical pregnancy.

In an embodiment, the female subject is undergoing hormone stimulation. In an alternative embodiment, the female subject is experiencing a natural ovulation cycle.

The first knowledge base of data may also come from multiple subjects or cohorts of subjects with known outcome of pregnancy.

The determination of the concentrations or levels of CSF3 and/or CSF3R enables establishment of a diagnostic rule based on the concentrations of the biomarkers relative to controls. Alternatively, the diagnostic rule is based on the application of a statistical and machine learning algorithm. Such an algorithm uses relationships between biomarkers and implantation outcome observed in training data (with known outcome status) to infer relationships which are then used to predict the status of an implantation even with unknown status. An algorithm is employed which provides an index of probability that a subject will become pregnant and, once pregnant, the likely status of the pregnancy in terms of a clinical pregnancy or preclinical pregnancy. The algorithm performs a multivariate analysis function.

Thus disclosed is a diagnostic rule based on the application of statistical and machine learning algorithms. Such an algorithm uses the relationships between CSF3 and/or CSF3R and implantation outcome status observed in training data (with known implantation status) to infer relationships which are then used to predict the status of subjects with unknown status. Practitioners skilled in the art of data analysis recognize that many different forms of inferring relationships in the training data may be used without materially changing the present invention.

The present description contemplates the use of a knowledge base of training data comprising levels of CSF3 and/or CSF3R from a subject with a known implantation outcome to generate an algorithm which, upon input of a second knowledge base of data comprising levels of the same biomarkers from a subject with an unknown implantation outcome likelihood, provides an index of probability that predicts the probable outcome.

The "subject" is a human female. In an embodiment, the human female is in a selected within child bearing age range.

The term "training data" includes knowledge of levels of biomarkers relative to a control. This is the first knowledge base. A "control" includes levels of biomarkers in a subject or cohort of subjects of known implantation success or failure status or may be a statistically determined level based on trials. The term "levels" also encompasses ratios of levels of biomarkers.

The "training data" also include the concentration of CSF3 or level of CSF3R. The data may comprise information on an increase or decrease in these biomarkers. Additional biomarkers may also be used such as but not limited to VEGF, IL-6, IL-8, IL-17A, CRP, PlGF, sFlt-1 and sGP130.. Progesterone may also be assayed. Similar to the foregoing embodiments in relation to CSF3 and CSF3R, elevation of CSF3 (or reduction in CSF3R) together with an elevation in one or more of VEGF, IL-6, IL-8, IL-17A, CRP, PlGF, sFlt-1 and/or sGP130 is indicative of a poor likelihood of a successful pregnancy. In an embodiment, any one of CSF3, CSF3R, IL-17A and/or PIGF may be assayed in a stand alone assay.

The present disclosure, but not the invention as claimed, further contemplates a panel of agents for stratification of a female subject undergoing assisted reproduction, the panel comprising agents which bind specifically to CSF3 and/or CSF3R, which are used to determine levels of CSF3 and/or CSF3R and then subjecting the levels to an algorithm generated from a first knowledge base of data comprising the levels of the same biomarkers from a subject of known status with respect to embryo implantation wherein the algorithm provides an index of probability of the subject having or not having a successful pregnancy. A "successful" pregnancy is one leading to a clinical pregnancy. Subsequent events leading to a miscarriage are not contemplated in the definition of a "successful" pregnancy.

The levels or concentrations of CSF3 and/or CSF3R in an assayed female subject provide the input test data referred to herein as a "second knowledge base of data". The second knowledge base of data either is considered relative to a control or is fed into an algorithm generated by a "first knowledge base of data" which comprise information of the levels of the biomarkers in a subject with a known implantation outcome. The second knowledge base of data is from a subject or cohort of subjects of unknown status with respect to an embryo implantation event. The output of the algorithm is a probability or likelihood factor, referred to herein as an index of probability, of a subject having a successful pregnancy in terms of ultimately achieving a clinical pregnancy. Levels may be determined in one type of fluid sample (e.g. uterine lavage or serum) or from two or more sources (e.g. uterine lavage and serum).

The agents which "specifically bind" to CSF3 or CSF3R generally include an immunointeractive molecule such as an antibody or hybrid, derivative including a recombinant or modified form thereof or an antigen-binding fragment thereof. The agents may also be a receptor or other ligand. These agents assist in determining the level of the biomarkers.

Hence, the present invention can further involve the use of a panel of immobilized ligands to CSF3 and/or CSF3R. The panel may comprise ligands to either or both biomarker. Such ligands include antibodies to CSF3 and/or CSF3R.

Still another aspect of the present description, not being part of the invention as claimed, contemplates a composition or kit for stratifying a female subject with respect to outcome of assisted reproduction, the kit comprising a composition of matter comprising one or more ligands to CSF3 and/or CSF3R; the kit further comprising reagents to facilitate determination of the concentration of CSF3 and/or CSF3R binding to a ligand. In use, the kit facilitates the determination of biomarkers. The levels of CSF3 or CSF3R or ratios of levels of CSF3 and CSF3R are then compared to a control or subjected to an algorithm generated from a first knowledge base of data comprising the levels of the same biomarkers from a subject or cohort of subjects of known status with respect to implantation outcome wherein the algorithm provides an index of probability of the subject having or not having the particular outcome. In an embodiment, the outcome is pregnancy or no pregnancy. In other embodiment the outcome is clinical pregnancy or preclinical pregnancy.

The ligands, such as antibodies specific to each of the biomarkers, enable the quantitative or qualitative detection or determination of the level of CSF3 and/or CSF3R. Reference to "level" includes concentration as weight per volume, activity per volume or units per volume or other convenient representative as well as ratios of levels. The "level" may be the extent of CSF3-mediated signaling and hence downstream indicators of CSF3-mediated signaling or CSF3R activity may be measured.

The "sample" includes a uterine sample such as a uterine lavage or aspirate. A uterine lavage provides the most consistency of data. Notwithstanding, the assay may be modified to use blood, plasma or serum, ascites, lymph fluid, tissue exudate or urine. Alternatively, the sample is a tissue sample which is biochemically examined.

Identifying levels of CSF3 and/or CSF3R in subjects undergoing an assisted reproduction technology protocol is useful in stratifying the outcome of the implantation. A person of ordinary skill in the art, based on the disclosure herein, can also identify additional biomarkers which may provide improved selectivity and sensitivity.

As indicated above, the "ligand" or "binding agent" and like terms, refers to any compound, composition or molecule capable of specifically or substantially specifically (that is with limited cross-reactivity) binding to an epitope on the biomarker. The "binding agent" generally has a single specificity. Notwithstanding, binding agents having multiple specificities for two or more biomarkers are also contemplated herein. The binding agents (or ligands) are typically antibodies, such as monoclonal antibodies, or derivatives or analogs thereof, but also include, without limitation: Fv fragments; single chain Fv (scFv) fragments; Fab' fragments; F(ab')2 fragments; humanized antibodies and antibody fragments; camelized antibodies and antibody fragments; and multivalent versions of the foregoing. Multivalent binding reagents also may be used, as appropriate, including without limitation: monospecific or bispecific antibodies; such as disulfide stabilized Fv fragments, scFv tandems [(scFv)₂ fragments], diabodies, tribodies or tetrabodies, which typically are covalently linked or otherwise stabilized (i.e. leucine zipper or helix stabilized) scFv fragments. "Binding agents" also include aptamers, as are described in the art.

Methods of making antigen-specific binding agents, including antibodies and their derivatives and analogs and aptamers, are well-known in the art. Polyclonal antibodies can be generated by immunization of an animal. Monoclonal antibodies can be prepared according to standard (hybridoma) methodology. Antibody derivatives and analogs, including humanized antibodies can be prepared recombinantly by isolating a DNA fragment from DNA encoding a monoclonal antibody and subcloning the appropriate V regions into an appropriate expression vector according to standard methods. Phage display and aptamer technology is described in the literature and permit *in vitro* clonal amplification of antigen-specific binding reagents with very affinity low cross-reactivity. Phage display reagents and systems are available commercially, and include the Recombinant Phage Antibody System (RPAS), commercially available from Amersham Pharmacia Biotech, Inc. of Piscataway, New Jersey and the pSKAN Phagemid Display System, commercially available from MoBiTec, LLC of Marco Island, Florida. Aptamer technology is described for example and without limitation in US Patent Nos. 5,270,163; 5,475,096; 5,840,867 and 6,544,776.

ECLIA, ELISA, Luminex LabMAP and Bioplex immunoassays are examples of suitable assays to detect levels of the biomarkers. In one example a first binding reagent/antibody is attached to a surface and a second binding reagent/antibody comprising a detectable group binds to the first antibody. Examples of detectable-groups include, for example and without limitation: fluorochromes, enzymes, epitopes for binding a second binding reagent (for example, when the second binding reagent/antibody is a mouse antibody, which is detected by a fluorescently-labeled anti-mouse antibody), for example an antigen or a member of a binding pair, such as biotin. The surface may be a planar surface, such as in the case of a typical grid-type array (for example, but without limitation, 96-well plates and planar microarrays) or a non-planar surface, as with coated bead array technologies, where each "species" of bead is labeled with, for example, a fluorochrome (such as the Luminex technology described in U. S. Patent Nos. 6,599, 331,6, 592,822 and 6,268, 222), or quantum dot technology (for example, as described in U. S. Patent No. 6,306. 610). Such assays may also be regarded as laboratory information management systems (LIMS).

In the bead-type immunoassays, the Luminex LabMAP system can be utilized. The LabMAP system incorporates polystyrene microspheres that are dyed internally with two spectrally distinct fluorochromes. Using precise ratios of these fluorochromes, an array is created consisting of 100 different microsphere sets with specific spectral addresses. Each microsphere set can possess a different reactant on its surface. Because microsphere sets can be distinguished by their spectral addresses, they can be combined, allowing up to 100 different analytes to be measured simultaneously in a single reaction vessel. A third fluorochrome coupled to a reporter molecule quantifies the biomolecular interaction that has occurred at the microsphere surface. Microspheres are interrogated individually in a rapidly flowing fluid stream as they pass by two separate lasers in the Luminex analyzer. High-speed digital signal processing classifies the microsphere based on its spectral address and quantifies the reaction on the surface in a few seconds per sample.

As used herein, "immunoassay" refers to immune assays, typically, but not exclusively sandwich assays, capable of detecting and quantifying a desired biomarker, namely CSF3 and/or CSF3R.

Data generated from an assay to determine uterine (or other sample)levels of CSF3 and/or CSF3R, can be used to determine the likelihood of progression of an embryo implantation event to clinical pregnancy in a female subject. The input of data comprising the levels of CSF3 and/or CSF3R is compared with a control or is put into the algorithm which provides a likelihood value of a successful pregnancy. By "successful pregnancy" as indicated above, refers to a pregnancy. Further outcomes such as a clinical or preclinical pregnancy can also be discriminated. An assisted reproductive treatment regime can also be monitored by the subject prognostic assay.

As described above, methods are taught herein for stratifying a female subject for expected outcome of embryo implantation by determining levels of CSF3 and/or CSF3R and using these levels as second knowledge base data in an algorithm generated with first knowledge base data or levels of the same biomarkers in patients with a known implantation outcome. By "velocity" it is meant the change in the concentration of the biomarker in a patient's sample over time.

The term "control sample" includes any sample that can be used to establish a first knowledge base of data from subjects with a known embryo implantation outcome.

The method of the subject invention may be used in the determination of the likely outcome of a cycle of assist reproductive technology (i.e. pregnancy or not pregnancy). The present invention may also be used to monitor the progression of a pregnancy and to monitor whether a particular treatment is effective or not pre-implantation. In particular, the method can be used to confirm the present or absence of conditions in the endometrium to be most receptive for implantation to occur and proceed to a clinical pregnancy.

As indicated above, antibodies may be used in any of a number of immunoassays which rely on the binding interaction between an antigenic determinant of the biomarker and the antibodies. Examples of such assays are radioimmunoassay, enzyme immunoassays (e.g. ECLIA, ELISA), immunofluorescence, immunoprecipitation, latex agglutination, hemagglutination and histochemical tests. The antibodies may be used to detect and quantify the level of the biomarker in a sample in order to determine endometrium receptivity leading to a level of likelihood of a successful pregnancy.

In particular, the antibodies of the present invention may also be used in immunohistochemical analyses, for example, at the cellular and subcellular level, to detect a biomarker, to localize it to particular cells and tissues, and to specific subcellular locations, and to quantitate the level of expression.

Cytochemical techniques known in the art for localizing antigens using light and electron microscopy may be used to detect the biomarker. Generally, an antibody of the present invention may be labeled with a detectable substance and a biomarker protein may be localized in tissues and cells based upon the presence of the detectable substance. Examples of detectable substances include, but are not limited to, the following : radioisotopes (e.g. ³H, ¹⁴C ³⁵S, ¹²⁵I, ¹³¹I), fluorescent labels (e.g. FITC, rhodamine, lanthanide phosphors), luminescent labels such as luminol; enzymatic labels (e.g. horseradish peroxidase, betagalactosidase, luciferase, alkaline phosphatase, acetylcholinesterase), biotinyl groups (which can be detected by marked avidin e.g. streptavidin containing a fluorescent marker or enzymatic activity that can be detected by optical or calorimetric methods), predetermined polypeptide epitopes recognized by a secondary reporter (e.g. leucine zipper pair sequences, binding sites for secondary antibodies, metal binding domains; epitope tags). In some embodiments, labels are attached *via* spacer arms of various lengths to reduce potential steric hindrance. Antibodies may also be coupled to electron dense substances, such as ferritin or colloidal gold, which are readily visualized by electron microscopy.

The antibody or sample may be immobilized on a carrier or solid support which is capable of immobilizing cells, antibodies etc. For example, the carrier or support may be nitrocellulose, or glass, polyacrylamides, gabbros, and magnetite. The support material may have any possible configuration including spherical (e.g. bead), cylindrical (e.g. inside surface of a test tube or well, or the external surface of a rod), or flat (e.g. sheet, test strip) Indirect methods may also be employed in which the primary antigen-antibody reaction is amplified by the introduction of a second antibody, having specificity for the antibody reactive against biomarker protein. By way of example, if the antibody having specificity against CSF3 or CSF3R is a rabbit IgG antibody, the second antibody may be goat anti-rabbit gamma-globulin labeled with a detectable substance as described herein.

Where a radioactive label is used as a detectable substance, the biomarker may be localized by radioautography. The results of radioautography may be quantitated by determining the density of particles in the radioautographs by various optical methods.

The methods of the present invention described herein may also be performed using microarrays, such as oligonucleotide arrays, cDNA arrays, genomic DNA arrays, or antibody arrays.

In an embodiment, the method of the present invention involves the detection of mRNA encoding CSF3 or CSF3R and to determine the level of biomarkers based on level of expression. Those skilled in the art can construct nucleotide probes for use in the detection of mRNA sequences encoding the biomarker(s) in samples. Suitable probes include nucleic acid molecules based on nucleic acid sequences encoding at least five sequential amino acids from regions of the biomarker, in particular they comprise 15 to 30 nucleotides. A nucleotide probe may be labeled with a detectable substance such as a radioactive label which provides for an adequate signal and has sufficient half-life such as ³²P, ³H, ⁴⁴C or the like. Other detectable substances which may be used include antigens that are recognized by a specific labeled antibody, fluorescent compounds, enzymes, antibodies specific for a labeled antigen, and luminescent compounds. An appropriate label may be selected having regard to the rate of hybridization and binding of the probe to the nucleotide to be detected and the amount of nucleotide available for hybridization. Labeled probes may be hybridized to nucleic acids on solid supports such as nitrocellulose filters or nylon membranes as generally described in Sambrook et al, Molecular Cloning, A Laboratory Manual. (2nd ed.), 1989. The nucleic acid probes may be used to detect gene transcripts that encode CSF3 or CSF3R which provides an indication of tier level. In an embodiment, the probes are used in the stratification of a female subject by specifically determining the level of expression of a biomarker.

The probe may be used in hybridization techniques to detect expression of genes that encode biomarker proteins. The technique generally involves contacting and incubating nucleic acids (e.g. mRNA) obtained from a uterine sample from female subject or other cellular source with a probe under conditions favorable for the specific annealing of the probes to complementary sequences in the nucleic acids. After incubation, the non-annealed nucleic acids are removed, and the presence of nucleic acids that have hybridized to the probe if any are detected.

The detection of mRNA may involve converting the mRNA to cDNA and/or the amplification of specific gene sequences using an amplification method such as polymerase chain reaction (PCR), followed by the analysis of the amplified molecules using techniques known to those skilled in the art. Suitable primers can be routinely designed by one of skill in the art.

Hybridization and amplification techniques described herein may be used to assay qualitative and quantitative aspects of expression of genes encoding the biomarker. For example, RNA may be isolated from a cell type or tissue known to express a gene encoding the biomarker, and tested utilizing the hybridization (e.g. standard Northern analyses) or PCR techniques referred to herein.

The primers and probes may be used in the above described methods *in situ* i.e. directly on tissue sections (fixed and/or frozen) of patient tissue obtained from uterine biopsies.

The present invention provides a method of stratifying a female subject with respect to the likelihood of a particular outcome of embryo implantation comprising:
(a) providing a fluid sample from the subject;
(b) extracting nucleic acid comprising mRNA from a sample encoding CSF3 and/or CSF3R;
(c) amplifying the extracted mRNA using the polymerase chain reaction;
(d) determining the level of mRNA encoding CSF3 or CSF3R; and
(e) subjecting the levels of transcript to an algorithm which provides an index of probability of the likely outcome of embryo implantation. A "fluid sample" includes in one embodiment, uterine lavage. In another embodiment, it is blood, plasma, serum, ascites, lymph fluid, tissue exudate or urine. In an embodiment, biomarker levels are determined and subject to multivariate analysis in combination with BMI and/or age of the patient.

The methods described herein may be performed by utilizing pre-packaged diagnostic kits comprising the necessary reagents to perform any of the methods of the invention. For example, the kits may include at least one specific nucleic acid or antibody described herein, which may be conveniently used, e.g. in clinical settings, to screen and diagnose patients and to screen and identify those individuals with an endometrium receptive for successful implantation of an embryo. The kits may also include nucleic acid primers for amplifying nucleic, acids encoding the biomarker in the polymerase chain reaction. The kits can also include nucleotides, enzymes and buffers useful in the method of the invention as well as electrophoretic markers such as a 200 bp ladder. The kit also includes detailed instructions for carrying out the methods of the present invention.

The above aspects further apply to measuring CSF3 or CSF3R in combination with one or more of VEGF, IL-6, IL-8, IL-17A, CRP, PlGF, sFlt-1 and/or sGP130. Progesterone can also be assayed. Elevation of any one of more of VEGF, IL-6, IL-8, IL-17A, CRP, PlGF, sFlt-1 and/or sGP130 in combination with CSF3 or CSF3R is indicative of a poor likelihood of a successful pregnancy outcome. In terms of stand alone assays are concerned, any one of CSF3, CSF3R, IL-17A and/or PIGF may be assayed. Elevated of CSF3, IL-17A and/or PIGF is indicative of a poor pregnancy likelihood.

Enabled herein is an algorithm-based screening assay to screen samples from female subjects undergoing an assisted reproduction technology protocol. Generally, input data are collected based on levels of CSF3 and/or CSF3R (or levels of expression of genes encoding CSF3 and/or CSF3R) and subjected to an algorithm to assess the statistical significance of any elevation or reduction in levels which information is then output data. Computer software and hardware for assessing input data are encompassed by the present invention.

Another aspect of the present invention contemplates a method of treating a female subject undergoing an assisted reproduction technology protocol, the method comprising administering to the subject an agent which antagonizes CSF3 activity or normalizes levels of CSF3 or CSF3R activity. Such an agent includes an agonist of CSF3R. It may not be necessary to totally inhibit CSF3 activity but to reduce the level of CSF3 activity to nondetrimental levels. This is regarding "normalizing" CSF3 activity or CSF3R levels.

The present invention further provides the use of an antagonist of CSF3 or an agonist of CSF3R in the manufacture of a medicament to facilitate a successful pregnancy.

Hence, contemplated herein is the use of an antagonist of CSF3 or agonist of CSF3R as an adjuvant to improve implantation rate in a female subject. As indicated above, CSF3, for example, may be inhibited to zero activity or inhibited to a level which equates to levels in a normal subject who experiences a successful pregnancy. Hence, complete inhibition of CSF3 or normalization of levels of CSF3 may be employed to effect a successful pregnancy outcome. Similarly, a CSF3R agonist may be used to elevate CSF3R which is otherwise downregulated due to a negative feedback mechanism by excess CSF3.

A pharmaceutical composition is thus contemplated herein comprising an antagonist of CSF3 or an agonist of CSF3R, the pharmaceutical composition further comprising one or more pharmaceutically acceptable carriers, excipients and/or diluents.

The terms "anatomist", "agonist", "medicament", "active", "drug", "medicine", "compound" and "therapeutic" may be used interchangeably herein to refer to a substance that induces a desired pharmacological and/or physiological effect including normalizing CSF3 signaling activity or the effects of CSF3 signaling activity. The pharmacological and/or physiological effect includes inhibiting the negative feedback effect of CSF3 on CSF3R. The terms also encompass pharmaceutically acceptable and pharmacologically active forms thereof, including salts. The desired effect is the inhibition or normalization of CSF3 activity preventing reduction in CSF3R levels.

Hence, the agents contemplated herein include CSF3 signaling modulators which encompass molecules which can downregulate CSF3 or upregulate CSF3R.

The terms "treating" and "treatment" as used herein refer to therapeutic treatment. The treatment results in an improved likelihood of a successful pregnancy.

The agent may be a proteinaceous or non-proteinaceous (i.e. a non-protein chemical) molecule. The term "agent" may also be described as an antagonist, agonist, medicament, active drug, therapeutic, medicine, compound or other molecule having an ability to normalize CSF3 signaling. Examples include antibodies to CSF3 or a soluble or mutated form of CSF3R or an oligonucleotide or microRNA which downregulates a gene or mRNA encoding CSF3.

In an embodiment, the agent is soluble CSF3R or a mutated form thereof. The "mutated" form includes a portion of CSF3R which contains one or more amino acid substitutions, additions or deletions. Such mutated forms may increase serum half-life or the mutated soluble CSF3R or increase binding activity to CSF3 thus reducing the amount of CSF3 available to bind to a CSF3R.

In an embodiment, the agent is a non-functional form of CSF3 which can bind to CSF3R without inducing CSF3 signaling. Such non-functional forms of CSF3 generally contain single or multiple amino acid substitutions, additions or deletions to a portion of CSF3 required for CSF3 signaling but which does not prevent it from binding to CSF3R/ Such a mutated CSF3 thus prevents active CSF3 from inducing signaling.

In an embodiment, the agent is an antibody specific for CSF3. The antibody any be a human antibody or a deimmunized or humanized form of a non-human antibody.

The terms "antibody" and "antibodies" include polyclonal and monoclonal antibodies and all the various forms derived from monoclonal antibodies, including but not limited to, full-length antibodies (e.g. having an intact Fc region); antigen-binding fragments, including for example, Fv, Fab, Fab' and F(ab')₂ fragments; and antibody-derived polypeptides produced using recombinant methods such as single chain antibodies. The terms "antibody" and "antibodies" as used herein also refer to human antibodies produced, for example, in transgenic animals or through phase display, as well as chimeric antibodies and humanized antibodies. It also includes other forms of antibodies that may be therapeutically acceptable and antigen-binding fragments thereof, for example, single domain antibodies derived from cartilage marine animals or Camelidae, or from libraries based on such antibodies. In relation to the latter embodiment, the antibody may be an immunoglobulin new antigen receptor (IgNAR) as defined in International Patent Publication No. WO 2005/118629 derived from a cartilaginous marine animal such as a shark. Reference may also be made to Nuttal et al. (2003) Eur. J. Biochem 270:2543-3554.

The term "monoclonal antibody" is used herein to refer to an antibody obtained from a population of substantially homogenous antibodies. That is, the individual antibodies comprising the population are identical except for naturally occurring mutations that may be present in minor amounts. The modifier "monoclonal" as used herein therefore indicates the character of the antibody as being obtained from a substantially homogenous population of antibodies, and is not used to indicate that the antibody was produced by a particular method. For example, monoclonal antibodies in accordance with the present invention may be made by the hybridoma method described by Kohler and Milstein (1975) Nature 256:495-499, or may be made by recombinant DNA methods (such as described in US Patent No. 4,816,567). Monoclonal antibodies may also be isolated from phage antibody libraries using the techniques described in Clackson et al. (1991) Nature 352:624-628 or Marks et al. (1991) J. Mol. Biol. 222:581-597.

Chimeric antibodies may include antibodies to CSF3 comprising the heavy and light chain variable regions of mouse, rat or rabbit antibodies to CSF3 and human heavy and light chain constant domains.

The agent may be a high level antagonist of the activity of CSF3 or it may be a low level antagonist designed or selected to reduce to non-zero levels the activity of the targeted molecule. The aim is to "normalize" CSF3 signaling to reduce the adverse effects of CSF3 on the endometrium. These aspects further apply to normalizing levels of VEGF, IL-6, IL-8, IL-17A, CRP, PlGF, sFlt-1 and/or sGP130.

Yet another embodiment taught herein is an assay for determining the risk that a pregnant woman may undergo a miscarriage, the assay comprising determining the concentration of CSF3 in a fluid sample from the pregnant woman, wherein if the level of CSF3 is less than a normal level relative to a control, this is indicative of a higher incidence of miscarriage.

Aspects disclosed herein are further exemplified by the following non-limiting Examples.

### EXAMPLES

### Materials and Methods

### Natural Cycling Patient Cohort

Uterine lavage and tissue biopsy (where applicable) were collected as previously described (Hannan et al. (2010) Journal of proteome research 9(12):6256-64) from fertile women (n=19) with proven parity undergoing routine gynaecologic procedures, and primary idiopathic infertile women (n=18) undergoing dilatation and curettage. Women with tubal or ovarian abnormalities (e.g. blocked tubes, tubal cyst, ovarian cyst, amenorrhea and Polycystic Ovarian Syndrome) were excluded as was male factor infertility. In brief lavage collection was performed by slow infusion of 3.0mL of sterile saline into the uterine cavity, using an infant feeding tube; the uterine contents were aspirated using the same tube. Cellular debris and mucus were removed by centrifugation at 1000rpm and pipette aspiration of the lavage, which was aliquoted and stored at -80C prior to analysis. Tissue biopsy sample was submitted to an accredited pathology laboratory for dating based on Noyes criteria (Noyes et al. (1975) American journal of obstetrics and gynecology 122(2):262-3). Only those samples pathology dated as early secretory stage were included in this study.

### Assisted Reproduction Therapy Stimulated Cycle Cohort

Cohort of women undergoing stimulated IVF cycles, lavage was performed at the time of oocyte retrieval (hCG+2). All women underwent fresh embryo transfer in the same cycle and be classified idiopathic infertile with potential causes (e.g. PCOS, endometriosis, male factor and hydrosalpinges) having been eliminated. Patients were divided into three groups according to cycle outcome, i.e. pregnancy (n=4) no pregnancy (n=4), and pre-clinical pregnancy (n=4). A fourth group comprising fertile women undergoing IVF stimulation as egg donors (n=4) was also collected. A matched tissue biopsy sample was collected for each lavage.

### Immunoassay Analysis of Uterine Lavage

Initial analysis was made of lavage from the natural cycle cohort. All assays were performed using a Bioplex 200 instrument (Biorad Laboratories, Berkeley, CA, USA). The assays used were a Milliplex Human Cytokine panel (Merck Millipore, Billerica, MA, USA) for IL8 and CSF3, a Milliplex Soluble Cytokine Receptor panel for sFlt-1 and sGP130 (Merck Millipore), and a PIGF bead immunoassay from R&D systems (Minneapolis, MN, USA). All assays were performed on uterine lavage samples with no further dilution, and were performed according to manufacturer's instructions with an overnight sample incubation at +4°C.

### Immunohistochemical Detection of CSF3 and its Receptor in Uterine Tissue

Immunohistochemistry for CSF3 and its receptor CSF3R was performed using a goat polyclonal antibody to CSF3 (N-20, Santa Cruz Biotechnology, Dallas, TX, USA) and a mouse monoclonal antibody to CSF3R (clone S-1284, Abcam, Cambridge, MA, USA) at dilutions of 1:200 and 1:400v/v, respectively. A matched species non-specific IgG was used as a negative control for each antibody. Secondary detection antibodies were biotinylated horse anti-goat and horse anti-mouse (Vector Laboratories, Burlingame, CA, USA) respectively. Detection was performed using ABC vector stain (Vector Laboratories) in conjunction with DBA substrate (Glostrup, Dako, USA).

### Hormone dependent expression of CSF3R

ECC-1 cells whose origin was confirmed by genotype and tested mycoplasma free were raised to 80% confluence in DMEM/F12 (Gibco, Life Technologies, Carlsbad, CA, USA) containing 10% v/v fetal calf serum (FCS) (Gibco, Life Technologies) before being serum-starved for 24 hours in DMEM/F12 containing 0.5% w/v charcoal-stripped (CS) FCS (Gibco, Life Technologies). Cells were cultured in this medium for a further 24 hours with or without the addition of 10⁻⁸M estradiol 17β (E) (Sigma Aldrich, St. Louis, MO, USA) and then for a final 24 hour period with no hormone, E only or E plus 10⁻⁷M Medroxyprogesterone 17-acetate (MPA) [Sigma Aldrich]. Media were harvested at each 24 hour time point, centrifuged to remove cellular debris and snap frozen. On completion of the experiment, cells were harvested in RIPA buffer (50mM Tris, 150mM NaCl, 0.1% w/v SDS, 1% v/v Triton-X 100, 0.1% w/v Protease inhibitor cocktail) and frozen until analysis. Lysed cells were thawed and centrifuged to remove cellular debris.

### Primary Uterine Epithelial Culture

Uterine epithelial cells were isolated from proliferative phase endometrial biopsies from fertile women, and cultured as previously described (Paiva et al. (2011) Human reproduction 26(5):1153-62). The isolated epithelial cells were hormone-treated in identical manner to the cell line described above. Media and cell lysates were collected post-treatment and stored frozen prior to analysis.

### Total Protein Concentration

The total protein concentration of cell lysates was determined using BCA protein assay kit (Thermo Fisher). All samples were tested in triplicate at 3 dilutions. The absorbance was read at 560nm using an AgileCon plate reader (ACTGene Inc. Piscataway, NJ, USA) The mean concentration for each sample was calculated.

### Western blotting

Cell lysates (10ug) were separated by SDS-PAGE on mini-protean TGX 4-20% gradient acrylamide gels and transferred to 0.2µm PVDF membrane (Biorad, USA) for 7 minutes at 25V using a Trans-blot Turbo (Biorad). The membrane was blocked with 5% w/v non-fat milk in TBS/T20 and the blot probed with previously described (Layton et al. (1997) Growth factors 14(2-3):117-30) monoclonal antibody (LMH711) [Ludwig Institute for Cancer Research, Zurich, Switzerland] at a dilution of 2µg/mL. Bound antibody was detected using biotinylated horse anti-mouse IgG (Vector Laboratories) and ABC Vectastain (Vector Laboratories). Bands were visualized using Clarity (Trade Mark) Western ECL substrate (Biorad). The blot was imaged using a ChemiDoc (Trade Mark) MP (Biorad) and densitometric analysis comparing expression in E and E+P treated cells performed using beta-actin content visualized by HRP-conjugated anti-β-actin rabbit monoclonal antibody (13E5) [Cell Signalling Technology, Danvers, MA, USA] as a loading control.

### Statistical Analysis

Statistical analysis was performed using MedCalc (Trade Mark) and Graphpad Prism (Trade Mark) softwares. Comparison of analyte data for fertile v infertile natural cycling cohort was performed by Mann-whitney, and correlation analysis (spearman non-parametric) between age, protein concentration and individual markers was performed. Similar analysis was performed for the ratios of analytes. Analysis of the IVF cohort compared the three outcome groups (pregnancy, no pregnancy and preclinical pregnancy) by Kruskal-wallis with a post-hoc Dunn's analysis (p<0.05) to determine significance.

### EXAMPLE 1

### Natural Cycling Cohort

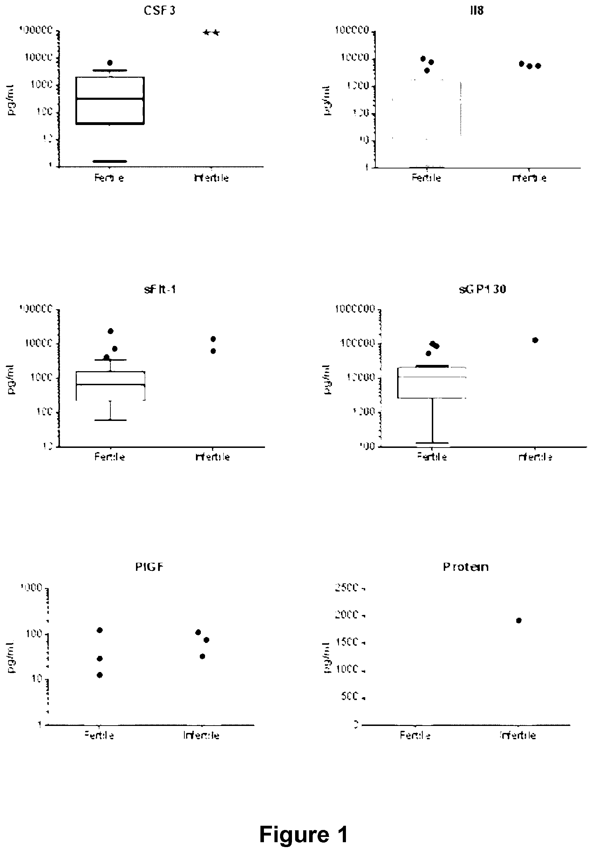

Uterine lavage and endometrial biopsy from 19 fertile and 18 primary idiopathic infertile women were staged as early secretory. There was no significant difference in age between the fertile (36.7 ± 4.0 years) and infertile (35.2 ± 5.3 years) groups. Detailed medical histories and pathology reports revealed a mixture of uterine pathologies and bleeding abnormalities among the patient cohort (Table 2). levels of biomarkers in uterine lavage are shown in Figure 1.

Analysis by Mann-Whitney test and ROC compared individual analyte concentrations in uterine lavage from fertile and infertile women during the early secretory phase of the cycle (Table 3). Among early secretory samples only CSF3 showed significant discrimination of fertile (mean = 1300+/- 435.8pg/mL) and infertile (mean = 3309+/-705.4pg/mL) women, by Mann-Whitney (p=0.006). No other markers showed significant discrimination between fertile and infertile women in the early secretory phase.

Spearman correlation analysis found no markers correlated with age among either the fertile or infertile women. Only sGP130 (r = 0.640, p= 0.003) and sFlt-1 (r = 0.553, p= 0.014) significantly correlated with total protein concentration in the fertile cohort and similarly both sGP130 (r=0.484, p=0.050) and sFlt-1 (r=0.742, p=0.001) were correlated with protein concentration in the infertile cohort. In the fertile group, significant correlation was seen between sGP130 with both CSF3 (r=0.490, p=0.033) and sFlt-1 (r=0.507, p=0.027) and while the former relationship was maintained (r=0.475, p=0.056) among the infertile women the latter was strengthened (r=0.718, p=0.002). Additional strong correlations were evident between CSF3 v IL8 (r=0.798, p=0.00007), and CSF3 v sFlt-1 (r=0.650, p=0.006) in the infertile women.

Given that all samples were collected in identical manner it is assumed that all samples will have been diluted to the same extent by the infused saline and regardless of the amount subsequently harvested. It is estimated that there is approximately 60µl of fluid

(Casslen (1986) The Journal of reproductive medicine 31(6):506-10) naturally present within the uterine cavity at hCG+2, thus infusion with 3ml of saline results in lavage diluted by a factor of 50.

### EXAMPLE 2

### Analysis of Lavage from Women undergoing ART

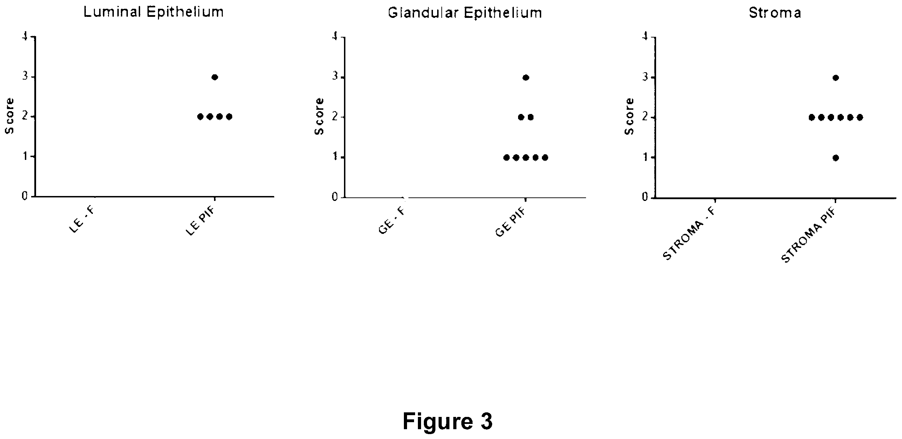

Concentrations of CSF3 in uterine lavage from women at hCG+2 in stimulated cycles were compared. Patients were divided into three groups according to cycle outcome: pregnancy, no pregnancy, and pre-clinical pregnancy, with a fourth group comprising fertile women undergoing IVF stimulation as egg donors. Concentrations of CSF3 differed significantly (Kruskal-Wallis p=0.0002) between groups being elevated in women who did not become pregnant (mean±SEM, 3447±89) compared to those who did achieve pregnancy (mean±SEM, 1245±269), preclinical pregnancy (mean±SEM, 1992±40) and the fertile stimulated egg donors (mean±SEM, 719±274) [Figure 3]. Analysis of individual pairings (Dunn's test) showed significant differences between 'no pregnancy' and both 'pregnancy' (p=0.041) and 'donor' (p = 0.025) groups.

### EXAMPLE 3

### Detection of CSF3 and its Receptor in Uterine tissue

Immunohistochemistry of CSF3 within early secretory phase tissues showed a general pattern of expression in luminal and glandular epithelium, and to some extent stromal expression, similar to that previously reported for mid-secretory tissue. There was, however, no clear difference in staining pattern of CSF3 between fertile and infertile women. Results are shown in Figure 2.

The CSF3 receptor revealed substantial staining in both luminal and glandular epithelium of fertile women during the early secretory phase of the cycle, while stromal staining was weak in comparison. However, among idiopathic infertile women, the epithelial expression of CSF3R was much reduced or even absent, while stromal staining was more intense.

Among the small IVF cohort of tissues sampled at hCG+2 there was a similar pattern of intense glandular epithelial staining in tissue from fertile egg donors undergoing IVF stimulation procedures and in women who went on to a successful pregnancy however in the women who did not become pregnant epithelial staining was markedly reduced or absent, though stromal staining was evident. Western blot analysis, using an antibody directed to CSF3R, of lysates of primary uterine epithelial cells revealed a band of approximately 89.2kDa. There are four known read-isoforms of the membrane bound CSF3R, (the canonical isoform 1 has Mw of 89.6kDa, isoform 2 85.1kDa, isoform 3 95.1kDa and isoform4 86.6kDa). The form of CSF3R present in the non-pregnant uterus has not been reported, though three isoforms are present in the placenta.

### EXAMPLE 4

### Effect of CSF3 on Endometrial Epithelial Cells Using xCelligence realtime Cell Analysis

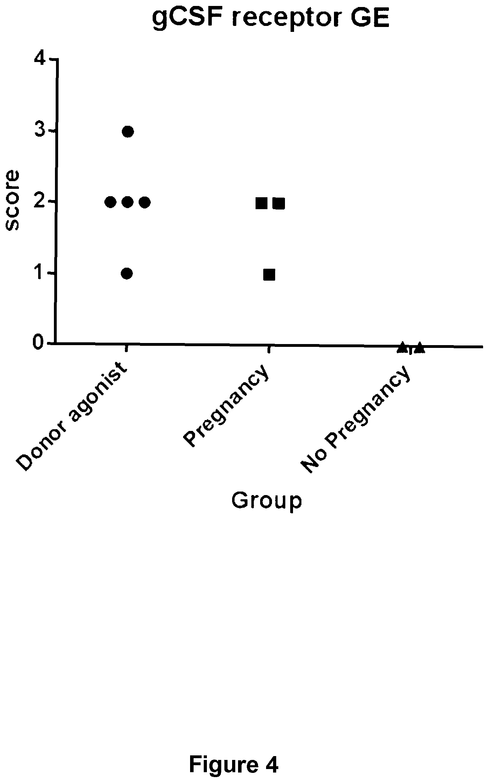

In Figure 4, both CSF3-G (glycosylated) and CSF3-NG (non-glycosylated) are observed to increase the adhesiveness of ECC1 cells in both chronic and acute treatment models with little dose response effect, however, the chronic treatment (24 hour prior exposure) with CSF3-G showing a diminished response, indicative that the cells have become less responsive to GSF3-G. This is a potentially significant result that GSF3-G falls away whereas CSF3-NG does not.

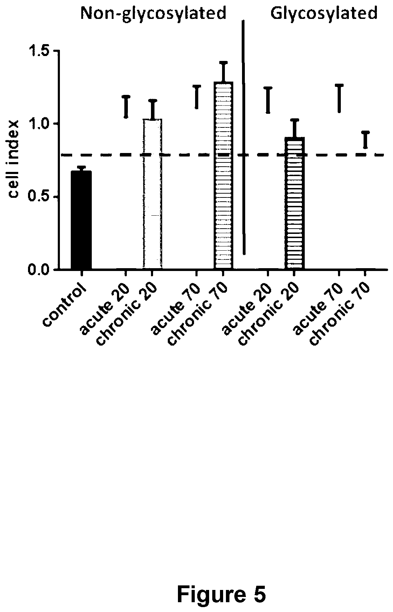

Proliferation experiments (Figure 5) showed both CSF3 forms increased proliferation of the cells, however, with CSF3-NG, the higher 70ng/mL concentration elicited a greater increase than 20ng/mL with the response to chronic treatment exceeding that of acute treatment. In contrast with CSF3-G the higher concentration (70ng/mL) resulted in a lesser increase in cell proliferation and in contrast to the CSF3-NG chronic exposure resulted in a reduced response to CSF3-G.

Experiments with the Ishikawa gland cell model found the acute treatments had little impact on either adhesion or proliferation. However chronic treatment elicited a trend to reducing adhesion, with both CSF3-G and CSF3-NG showing similar results at 70ng/mL, however, the 20ng/mL CSF3-NG showed a highly significant decrease in adhesion.

Proliferation of Ishikawa cells were significantly reduced by 70ng/mL CSF3-G compared with the 20ng/mL dose which increased proliferation. The reverse dose response effect was again seen with the CSF3-NG.

### EXAMPLE 5

### Effect of CSF3 on human Trophoblast Cells Using xCelligence realtime Cell Analysis

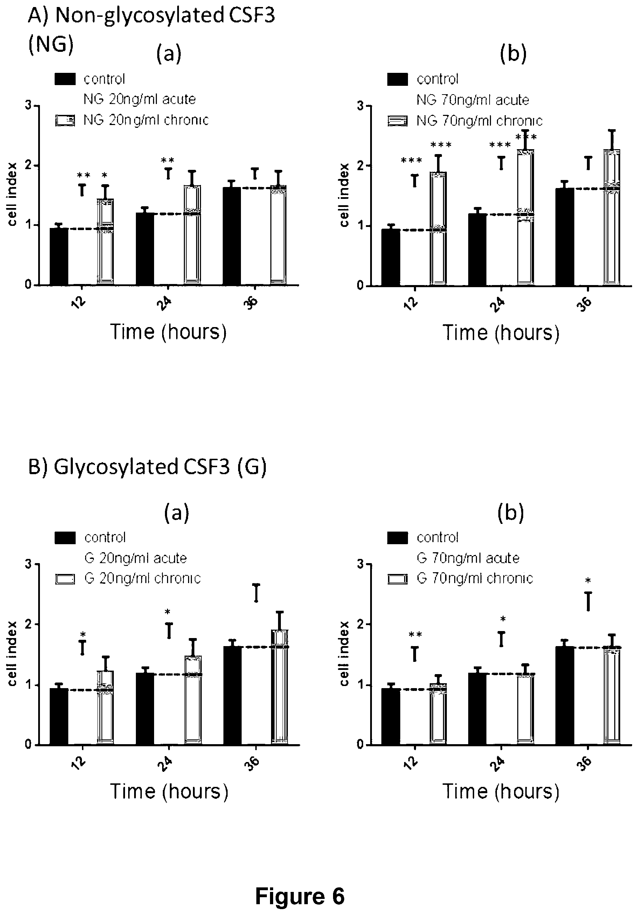

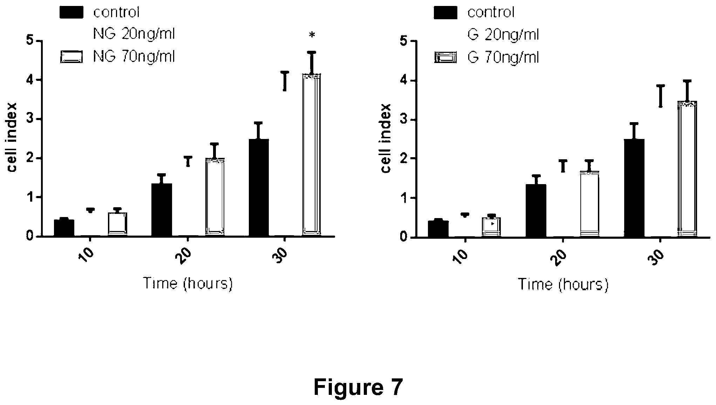

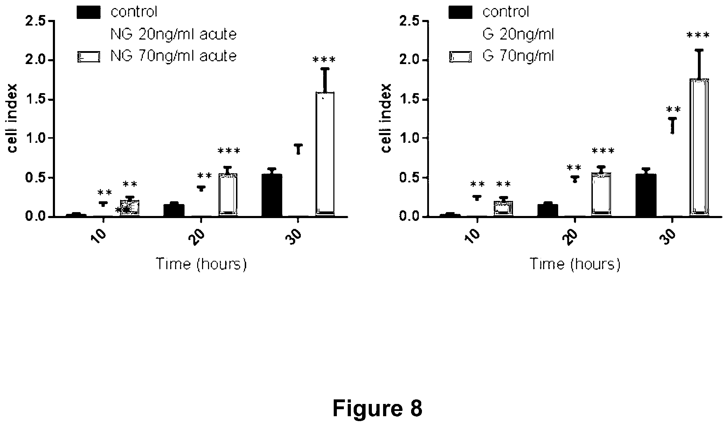

In Figure 6 HTR8 trophoblast cell line shows significantly increased invasion as a result of both CSF3-G and CSF3-NG inclusion in media, the response to 20ng/mL though less evident showed a similar trend to increasing invasion. Migration data showed a trend to increase migration of the Htr8 cell though only 70ng/mL CSF-NG after 30 hours showed significance. Data are also shown in Figures 7 and 8.

### EXAMPLE 6

### Role of CSF3

Synchronous development of the blastocyst and the endometrium is essential for successful establishment of pregnancy. The endometrium becomes receptive to implantation of the embryo for only a brief period of time during each menstrual cycle. A lack of appropriate endometrial development results in infertility, and to a lack of success with artificial reproductive technologies. Over the past decade, a number of laboratories have sought to identify markers of endometrial receptivity within the tissue or secretions of the uterine cavity. A number of genomic (Diaz-Gimeno et al. (2011) Fertility and sterility 95(1):50-60) and proteomic (Salamonsen et al. (2013) Fertility and sterility 99(4):1086-92; Scotchie et al. (2009) Reproductive sciences 16(9):883-93; Chen et al. (2009) Journal of proteome research 8(4):2032-44; Hannan *et al.* (2010) *supra)* analyses to identify receptivity biomarkers have been reported, in addition to single marker studies (Heng et al. (2011) Human reproduction 26(4):840-6; Krussel et al. (1999) Molecular human reproduction 5(5):452-8; Sherwin et al. (2002) The Journal of clinical endocrinology and metabolism 87(8):3953-60; Torry et al. (1996) Fertility and sterility 66(1):72-80; Hannan et al. (2011) Endocrinology 152(12):4948-56; Paiva *et al.* (2011) *supra;* Salmassi et al. (2005) Human reproduction 20(9):2434-40). The primary aim of many such studies has been to understand how endometrial receptivity develops and to define the embryo-maternal dialogue for implantation. Thus, a feature of studies to date has been the use of samples collected during the mid-secretory phase of a normal menstrual cycle (LH+5 to +7). It is acknowledged that genes or proteins regulated in this phase of the cycle may indeed reflect a receptive state thus providing the potential to identify a failure to achieve endometrial receptivity among idiopathic infertile women where other infertility causes have been excluded. However, there is the potential to develop a predictive test for application within an assisted reproduction therapy (ART) cycle to inform patient and clinician of the likelihood for successful implantation if an embryo transfer is planned. This is of particular relevance given increasing recognition that the endometrium of women during stimulated cycles is discordant with highly abnormal histology (Evans et al. (2014) Human reproduction update 20(6):808-21*;* Evans et al. (2012) PloS one. 7(12):e53098; Evans et al. (2013) Human reproduction 28(6):1610-9). Applicability to testing ART stimulation cycle is difficult as the 'mid-secretory' phase coincides with embryo transfer at approximately hCG+5 (when hCG is used for ovulation induction) potentially interfering with the transferred embryo and leaving insufficient time for laboratory analyses and reporting prior to embryo transfer. Additionally, it must be considered that, testing in a previous cycle is based on an assumption that the endometrium develops identically in every cycle: this has not been proven for natural cycles and further there is strong evidence that ovarian stimulation has considerable impact on the endometrium in stimulated cycles (Evans *et al.* 2014 *supra)* thus it cannot be assumed that similar receptivity is achieved if the patients stimulation protocol is revised.

Further many of these reports have utilized only very small patient numbers and often women with uterine pathologies or bleeding irregularities were excluded, when in reality they will comprise a good proportion of women for whom a receptivity test would be utilized.

In work leading to the present invention, it was investigated whether the biomarkers CSF3, IL8, PlGF, sGP130 and sFlt-1 could be used at the pre-receptive early secretory or hCG+2 point of natural and more particularly ART cycles respectively, to predict subsequent development of endometrial receptivity. The selected markers were initially examined for their discrimination of fertile and known primary idiopathic infertile women during the early secretory phase of their natural cycles. Those biomarkers able to discriminate at this phase were subsequently assessed in uterine lavage collected at hCG+2 (day of oocyte retrieval) from a cohort of infertile women undergoing IVF treatment with known embryo transfer outcomes; pregnancy, no pregnancy, preclinical pregnancy following same cycle transfer, alongside fertile egg donors.

Data obtained herein showed that only CSF3 could discriminate fertility status during the early secretory phase of the natural cycling women, and thus CSF3 determination was applied to a cohort collected at hCG+2 from women undergoing hormonal stimulation as part of an ART treatment cycle.

The regulation of CSF3 within uterine epithelial cells is not fully elucidated. In other cell types it is acknowledged that CSF3 exists in a negative feedback loop with its own receptor in immune associated cell types (Jilma et al. (2000) British journal of haematology 111(1):314-20). The presence of the CSF3 receptor is confirmed in in endometrial tissue, both in the early secretory phase of natural cycles and in ART women at hCG+2. Furthermore in women undergoing IVF there was also low or absent expression of the receptor associated with high levels of CSF3 and failure to achieve pregnancy. The receptor is abundant in epithelial cells (luminal and glandular) of fertile women, with little stromal expression, however, it was noted that among infertile women low epithelial expression of the receptor and an accompanying strong stromal expression is evident. Thus, it appears a negative feedback loop is indeed present among epithelial cells with the elevated CSF3 of infertile women resulting in decreased receptor expression. The mechanism of "switch on" for the stromal cell expression among the infertile women is unclear, though may potentially relate to local immune cell populations.

Functional studies of CSF3 on cultures of human endometrial epithelial cells and trophoblast cell line provide evidence of the impact that high CSF3 may have within the uterine cavity impacting fertility. The data examined both glycosylated (CSF3-G) and non-glycosylated forms of CSF3 (CSF3-NG), representative of lenograstrim (produced in Chinese hamster ovary cells) and filagastrim (produced in *E.coli*) the two major clinical approved products respectively. The data here haves demonstrated that the glycosylation state of the CSF3 impacts its functional effects, as indeed does the period of exposure to CSF3. The acute effect of CSF3 at two concentrations were examined: 70pg/mL representative of the mean concentration seen among infertile women, and 20ng/mL representative of that seen in fertile women, and the impact seen after a prior 24 hour exposure to the drug. Data here provide evidence that *in vivo* elevated CSF3 conditions exist in the uterine cavity prior to the implantation period, thus, it is likely that the chronic experiments are more closely aligned to that existing naturally. Adherence and proliferation of two endometrial epithelial cell line were examined, one representative of the luminal epithelium (ECCl) and the other of glandular epithelium (Ishikawa). Adherence effects were determined, an indicator of the ability endometrial cells to interact with an arriving trophoblast, as well as proliferation. Adherence was enhanced by increasing CSF3 concentrations in acute exposure, however, chronic exposure reduced this impact considerably. It was also of note that while CSF3-NG increased cell proliferation dose responsively, the glycosylated form showed the reverse relationship with higher concentrations showing reduced proliferation. While neither form is a true representation of human CSF3, the glycosylated form maybe considered a closer representation. With the gland epithelial model again the acute treatments showed little impact of CSF3, however, in the chronic exposure again the glycosylated and non-glycosylated forms showed reverse dose-response effects on proliferation with low-dose CSF3-G inhibiting proliferation and low dose CSF3-NG inhibiting adherence.

The impact in the chronic exposure belies the receptor loss resulting from CSF3 interaction, an interaction which will differ between glycosylated and non-glycosylated forms thus eliciting differing responses. These finding are highly significant in the clinical context with CSF3 being trialled as an adjuvant to improve pregnancy rates in ART. While uterine CSF3 has not previously been investigated with respect to endometrial based infertility, there its use clinically has been driven by an increasing interest in its relevance in follicular fluid and the granulosa cells in the ovary. CSF3 levels in follicular fluid are much higher in follicular fluid than serum (Salmassi *et al.* 2005 *supra).* However, in accordance with the present invention, CSF3 concentrations in uterine lavage are almost 10 times higher than those reported in follicular fluid. These localized high concentrations of CSF3 within the female reproductive tract suggest a local production and reproductive significance.

In summary, the data induce here that high CSF3 and low CSF3R in the uterus is associated with reduced fertility.

Demonstrated herein is not only the potential utility of CSF3 as a biomarker of female fertility, but more importantly defined some of its actions in the female reproductive tract including the influence of CSF3 and its receptor on potential embryo attachment within the uterine environment along with a potential influence on endometrial development as critical to endometrial function. Importantly, the clinical use of CSF3 as an adjuvant to improve implantation rates may be misguided and indeed contraindicated. It is proposed herein that neutralization of the excess CSF3 present in the endometrial secretions of women is a more effective path to improve pregnancy success.

**Table 2**

| ***The patient cohort with respect to fertility status, age, and presence of pathologies and bleeding disorders*** | | |
|---|---|---|
| | **Fertile** | **Infertile** |
| N= | 19 | 18 |
| **Mean Age** +/- **SD (years)** | 36.7±3.9 | 35.2±5.2 |

| **Women with Pathologies/Medical Conditions** | | |
|---|---|---|
| **N=** | 9 | 16 |

| **Women with individual pathologies/medical conditions** | | |
|---|---|---|
| **Irregular cycles** | 2 | |
| **Abdominal pain** | 2 | 1 |
| **Irregular Bleeding** | 4 | |
| **Menorrhagia** | 2 | |
| **Fimbrial cyst** | | 1 |
| **Cervical abnormality** | 3 | |

**Table 3**

| ***Mean (pg*/*mL), standard error of mean (SEM) and median of each biomarker in lavage collected from fertile and infertile cohorts staged as early secretory phase of cycle are shown. Statistical analysis using a non-parametric Mann-Whitney analysis: **p<0.01.*** | | | | | | | | |
|---|---|---|---|---|---|---|---|---|
| **Biomarker** | **Fertile** | | | **Infertile** | | | **Mann-** | **Significance P** |
| | **Mean** | **SEM** | **Median** | **Mean** | **SEM** | **Median** | **Whitney U** | |
| **CSF3** | 1300 | 435.8 | 330.3 | 3309 | 705.4 | 2838 | 82.00 | 0.0060^{∗∗} |
| **IL8** | 1503 | 667.7 | 327.4 | 1594 | 507.8 | 835.5 | 137.0 | 0.3101 |
| **PlGF** | 12.60 | 7.693 | 3.238 | 16.45 | 7.972 | 3.110 | 120.5 | 0.7873 |
| **sFlt-1** | 2581 | 1302 | 648.3 | 1991 | 891.5 | 436.1 | 150.0 | 0.7227 |
| **sGP130** | 21026 | 6942 | 11032 | 25037 | 8008 | 11603 | 138.0 | 0.4669 |

### EXAMPLE 7

### Modeling results for serum analysis

### Day 3 Transfers

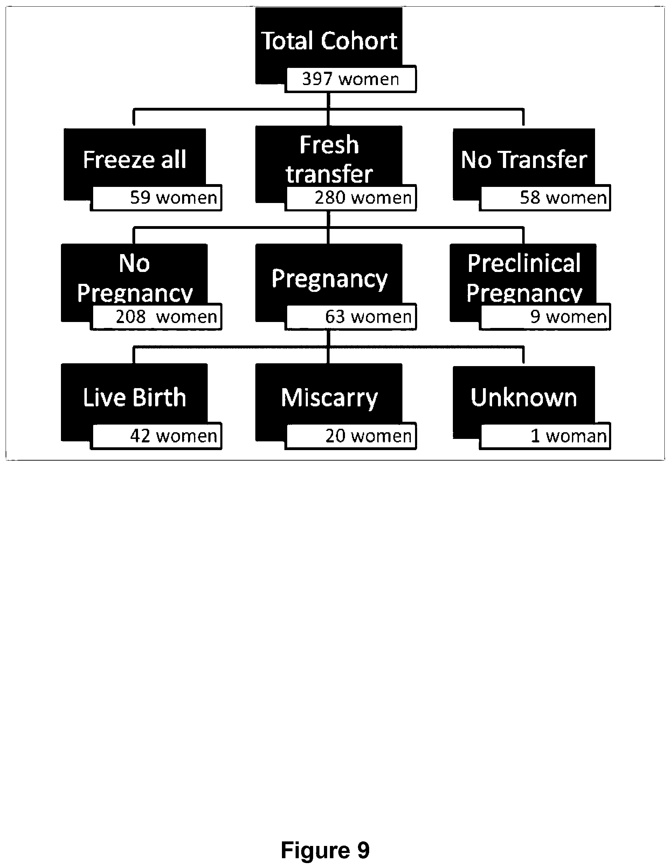

Day 3 Transfer outcomes were modelled in a two outcome model of 'pregnancy' (inclusive of live birth, miscarriage and preclinical) and 'no pregnancy' (inclusive of no pregnancy and preclinical/biochemical pregnancy) [Figure 9]. Example model below primarily used Age, E2/P4, IL-8/CRP, CSF3, progesterone, IL-6/IL-17A, no. eggs collected, endometrial thickness, Il6/IL8. There was a 99% correct classification of outcomes.

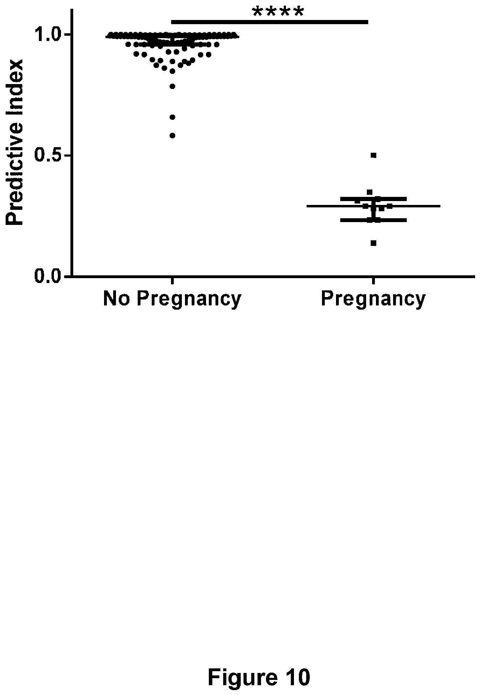

T-test found highly significant (p<0.0001) difference in predictive index between pregnancy and no pregnancy (Figure 10).

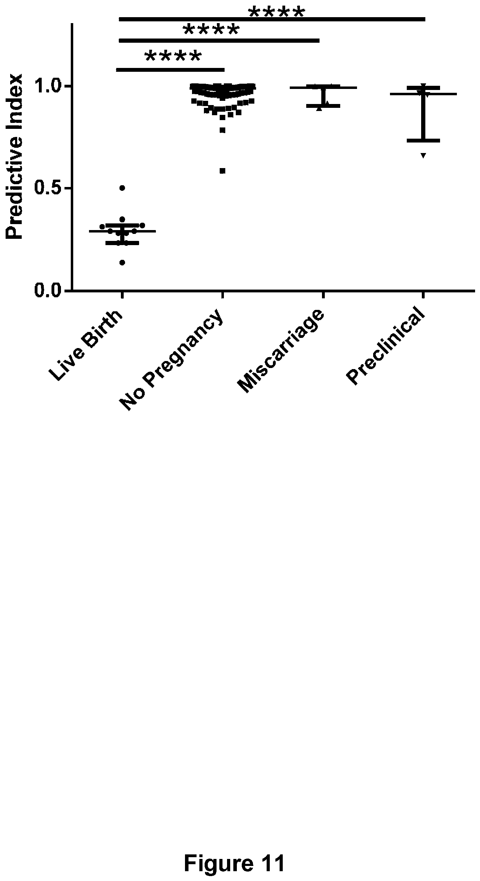

ANOVA with Tukey post-hoc test found highly significant (p<0.0001) difference in predictive index (Figure 11) arising from 2 way model between live birth and all three other categories (no pregnancy, miscarriage, preclinical/biochemical).

A second model was generated with 4 outcomes, live birth, miscarriage, preclinical/biochemical pregnancy and no pregnancy. There was 100% correct prediction of outcomes. The model used primarily age, IL-8/VEGF, P4 hCG+2-hCG0, CSF3/IL-6, IL-6/CRP, E2/P4, IL-8/IL-17A, CSF3, IL-8, CSF3/VEGF, IL-6/IL-8, endometrial thickness, IL-6, IL-17A/VEGF, eggs collected, Progesterone, IL-17A/CRP, IL-8/VEGF.

ANOVA with Tukey post-hoc test found significant difference in predictive index (Figure 11) arising from 2 way model between live birth and all three other categories (no pregnancy, miscarriage, preclinical/biochemical).

### Day 5 Transfers

Day 5 Transfer outcomes were modeled in a two outcome model of 'pregnancy' (inclusive of live birth, miscarriage and preclinical) and 'no pregnancy' (inclusive of no pregnancy and preclinical/biochemical pregnancy), total n=174 women. Example model below primarily used age, IL-6/IL-17A, progesterone, IL-8, CSF3, endometrial thickness. The model provided a 82% correct classification of samples.

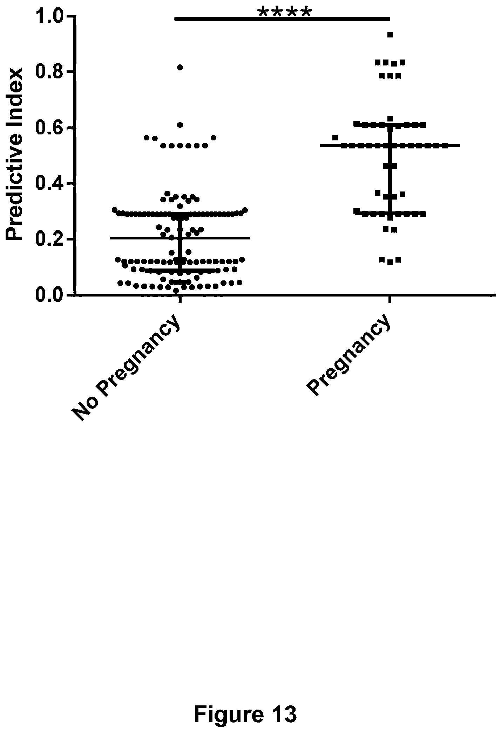

T-test found highly significant (p<0.0001) difference in predictive index between pregnancy and no pregnancy (Figure 13).

ANOVA with Tukey post-hoc test found highly significant (p<0.0001) difference in predictive index between live birth and all three other categories (no pregnancy, miscarriage, preclinical/biochemical). There was significant discrimination of women going on to a live birth as opposed to miscarriage, in addition to discrimination from no pregnancy and preclinical/biochemical pregnancy outcomes.

A second model was generated with 4 outcomes, live birth, miscarriage, preclinical/biochemical pregnancy and no pregnancy. There was 100% correct prediction of outcomes. Model used primarily embryo grade, CRP, age, IL-8, estrogen, VEGF, progesterone, no. eggs collected, total serum protein, CSF3/VEGF, CSF3/IL-6, IL-6/IL-8, egg fertilisation rate, CSF3, CSF3/IL-8, IL-8/VEGF, CSF3/IL-8, IL-17A/CRP, IL8/CRP, IL-17A/VEGF, BMI, IL-8/IL-17A, VEGF/CRP.

This model shows excellent prediction of pregnancy; significantly discriminating from miscarriage outcomes in addition to no pregnancy and preclinical outcomes.

### EXAMPLE 8

### IL-17A as a biomodal

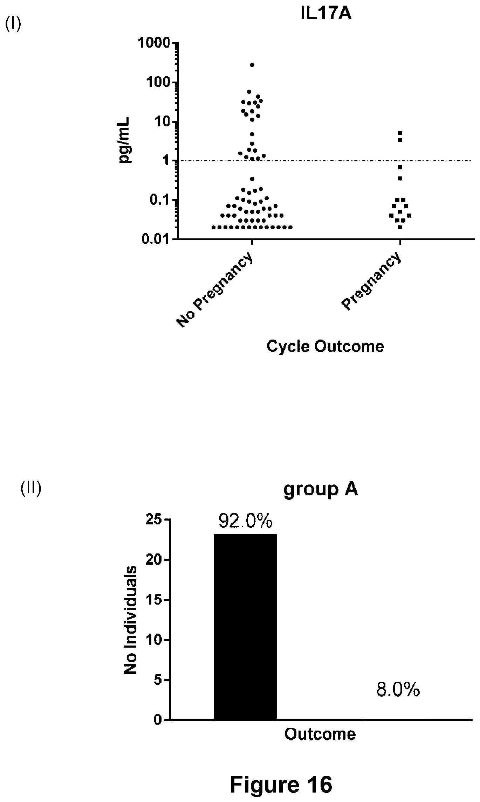

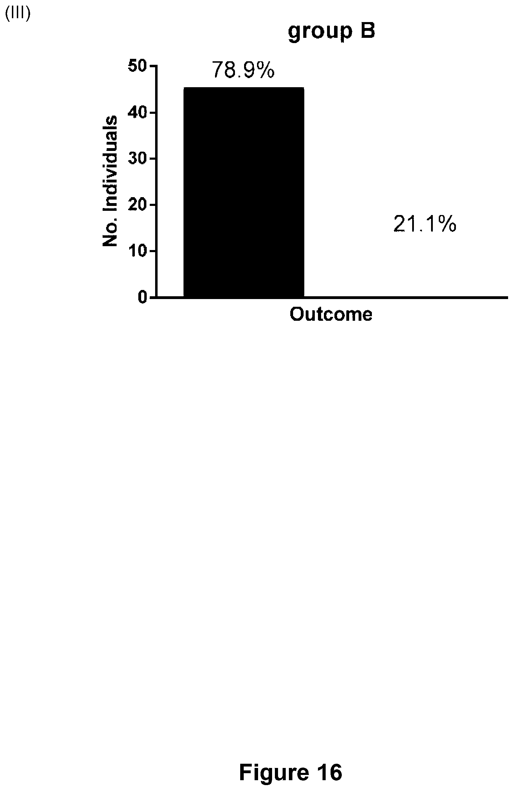

IL-17A in endometrial fluid shows a biomodal population. Population of women can be divided into a high (>1pg/mL) and a low (<1pg/mL) population, denoted as A and B respective in Figure 16. The pregnancy rate among population A is lower than B.

### EXAMPLE 9

### Assessment of biomarker

The association between biomarker levels and pregnancy outcome is shown in Table 4 and Table 5.

**Table 4**

| ***Summary of lavage concentration of individual biomarkers*** | | |
|---|---|---|
| | Fertile v Infertile (natural) | Pregnancy v No Pregnancy (IVF) |
| VEGF | Elevated infertile | Elevated no pregnancy |
| IL-6 | Elevated infertile | Elevated no pregnancy |
| IL-8 | Elevated infertile | Elevated no pregnancy |
| IL-17A | Not detectable | Elevated no pregnancy |
| CSF3 | Elevated Infertile | No difference |

**Table 5**

| ***Summary of serum concentration of individual biomarkers*** | |
|---|---|
| | Pregnancy v no Pregnancy (IVF) |
| VEGF | Elevated no pregnancy |
| IL-6 | Elevated no pregnancy |
| IL-8 | Elevated no pregnancy |
| IL-17A | Elevated no pregnancy |
| CSF3 | Elevated no pregnancy |
| CRP | Elevated no pregnancy |

### EXAMPLE 10

### Prediction of miscarriage

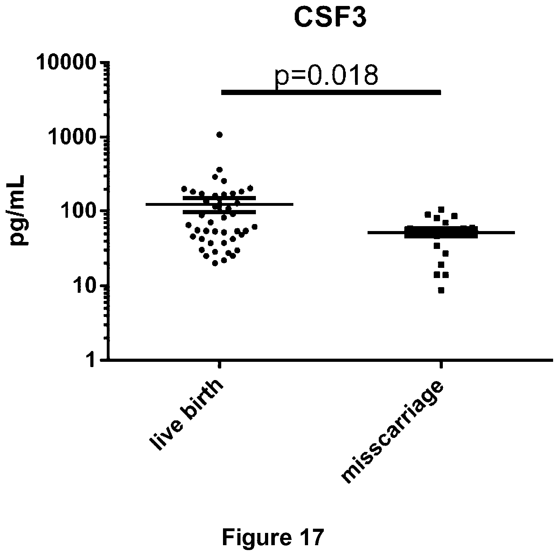

Figure 17 shows that levels of CSF3 can be used to predict the likelihood of miscarriage. Lower than normalized levels of CSF3 correlate to higher incidence of miscarriage.

Those skilled in the art will appreciate that the disclosure described herein is susceptible to variations and modifications other than those specifically described. It is to be understood that the disclosure contemplates all such variations and modifications. The disclosure also enables all of the steps, features, compositions and compounds referred to or indicated in this specification, individually or collectively, and any and all combinations of any two or more of the steps or features or compositions or compounds.

### BIBLIOGRAPHY

Casslen (1986) The Journal of reproductive medicine 31(6):506-10
Chen et al. (2009) Journal of proteome research 8(4):2032-44
Clackson et al. (1991) Nature 352:624-628
Diaz-Gimeno et al. (2011) Fertility and sterility 95(1):50-60
Evans et al. (2012) PloS one. 7(12):e53098
Evans et al. (2013) Human reproduction 28(6):1610-9
Evans et al. (2014) Human reproduction update 20(6):808-21
Hannan et al. (2010) Journal of proteome research 9(12):6256-64
Hannan et al. (2011) Endocrinology 152(12):4948-56
Heng et al. (2011) Human reproduction 26(4):840-6
Jilma et al. (2000) British journal of haematology 111(1):314-20
Kohler and Milstein (1975) Nature 256:495-499
Krussel et al. (1999) Molecular human reproduction 5(5):452-8
Layton et al. (1997) Growth factors 14(2-3):117-30
Macaldowie et al. (2013) Assisted reproductive technology in Australia and New Zealand 2011, Sydney: National Perinatal Epidemiology and Statistics Unit, the University of New South Wales, Australia
Marks et al. (1991) J. Mol. Biol. 222:581-597
Noyes et al. (1975) American journal of obstetrics and gynecology 122(2):262-3
Nuttal et al. (2003) Eur. J. Biochem 270:2543-3554
Paiva et al. (2011) Human reproduction 26(5):1153-62
Salamonsen et al. (2013) Fertility and sterility 99(4): 1086-92
Salmassi et al. (2005) Human reproduction 20(9):2434-40
Sambrook et al, Molecular Cloning, A Laboratory Manual. (2nd ed.), 1989
Scotchie et al. (2009) Reproductive sciences 16(9):883-93
Sherwin et al. (2002) The Journal of clinical endocrinology and metabolism 87(8):3953-60
Torry et al. (1996) Fertility and sterility 66(1):72-80

## Claims

1. A method for stratifying a human female subject with respect to expected outcomes of embryo implantation in an assisted reproduction technology protocol, the method comprising determining the levels of CSF3 and/or CSF3R in a fluid sample together with IL-17A and/or PlGF, wherein elevated levels of CSF3 relative to a control are indicative of a low likelihood of a successful pregnancy and reduced levels of CSF3R relative to a control are indicative of a low likelihood of a successful pregnancy and elevated levels of IL-17A and/or PlGF relative to controls are indicative of low likelihood of successful pregnancy and where normal CSF3 levels is indicative of high likelihood of a successful pregnancy outcome and normal levels of CSF3R are indicative of high likelihood of a successful pregnancy outcome and normal levels of IL-17A and/or PlGF are indicative of high likelihood of a successful pregnancy outcome, all relative to controls, wherein the fluid sample is selected from the list consisting of uterine lavage, blood, plasma or serum, ascites, lymph fluid, tissue exudate and urine.

2. The method of Claim 1 comprising determining the levels of CSF3 and/or CSF3R in a fluid sample together with IL-17A and PlGF.

3. The method of Claim 1 or Claim 2, wherein one or more of VEGF, IL-6, IL-8, CRP, sFlt-1 and sGP130 is also assayed, wherein an elevation of any one or more of these biomarkers relative to a control is indicative of a poor likelihood of a successful pregnancy outcome.

4. The method of Claim 3 wherein one or more of sFlt-1 and sGP130 is assayed, wherein an elevated level of the one or more of sFlt-1 and sGP130 relative to a control is indicative of a poor likelihood of a successful pregnancy outcome.

5. The method of any one of Claims 1 to 4, wherein the female subject is undergoing a hormone stimulated cycle.

6. The method of any one of Claims 1 to 4, wherein the female subject is undergoing a natural ovulation cycle.

7. The method of any one of Claims 1 to 6, wherein the fluid sample is a uterine lavage sample.

8. The method of any one of Claims 1 to 6, wherein the fluid sample is a blood, plasma or serum sample.

## Patentansprüche

1. Verfahren zum Stratifizieren eines menschlichen weiblichen Subjekts hinsichtlich erwarteter Ergebnisse der Embryoimplantation in einem Technologieprotokoll einer assistierten Reproduktion, wobei das Verfahren ein Bestimmen der Werte von CSF3 und/oder CSF3R in einer Fluidprobe zusammen mit IL-17A und/oder PIGF umfasst, wobei erhöhte CSF3-Werte relativ zu einer Kontrolle auf eine niedrige Wahrscheinlichkeit einer erfolgreichen Schwangerschaft hinweisen, und reduzierte CSF3R-Werte relativ zu einer Kontrolle auf eine niedrige Wahrscheinlichkeit einer erfolgreichen Schwangerschaft hinweisen und erhöhte IL-17A- und/oder PIGF-Werte relativ zu Kontrollen auf die niedrige Wahrscheinlichkeit der erfolgreichen Schwangerschaft hinweisen, und wobei normale CSF3-Werte auf eine hohe Wahrscheinlichkeit eines erfolgreichen Schwangerschaftsergebnisses hinweisen und normale CSF3R-Werte auf die hohe Wahrscheinlichkeit eines erfolgreichen Schwangerschaftsergebnisses hinweisen und normale IL-17A- und/oder PIGF-Werte auf die hohe Wahrscheinlichkeit eines erfolgreichen Schwangerschaftsergebnisses hinweisen, alle relativ zu den Kontrollen, wobei die Fluidprobe aus der Liste ausgewählt ist, die aus Uterusspülung, Blut, Plasma oder Serum, Aszites, Lymphflüssigkeit, Gewebeexsudat und Urin besteht.

2. Verfahren nach Anspruch 1, das das Bestimmen des CSF3- und/oder des CSF3R-Werts in einer Fluidprobe zusammen mit IL-17A und PIGF umfasst.

3. Verfahren nach Anspruch 1 oder 2, wobei einer oder mehrere von VEGF, IL-6, IL-8, CRP, sFlt-1 und sGP130 ebenso untersucht werden, wobei eine Erhöhung eines oder mehrerer dieser Biomarker relativ zu einer Kontrolle auf eine geringe Wahrscheinlichkeit eines erfolgreichen Schwangerschaftsergebnisses hinweist.

4. Verfahren nach Anspruch 3, wobei eines oder mehrere von sFlt-1 und sGP130 untersucht werden, wobei ein erhöhter Wert von einem oder mehreren von sFlt-1 und sGP130 relativ zu einer Kontrolle auf eine geringe Wahrscheinlichkeit eines erfolgreichen Schwangerschaftsergebnisses hinweist.

5. Verfahren nach einem der Ansprüche 1 bis 4, wobei das weibliche Subjekt einem stimulierten Hormonzyklus unterzogen wird.

6. Verfahren nach einem der Ansprüche 1 bis 4, wobei das weibliche Subjekt einem natürlichen Ovulationszylus unterzogen wird.

7. Verfahren nach einem der Ansprüche 1 bis 6, wobei die Fluidprobe eine Uterusspülungsprobe ist.

8. Verfahren nach einem der Ansprüche 1 bis 6, wobei die Fluidprobe eine Blut-, Plasma- oder Serumprobe ist.

## Revendications

1. Procédé de stratification d'un sujet humain de sexe féminin par rapport aux résultats attendus de l'implantation d'un embryon dans le cadre d'un protocole de technologie de procréation assistée, le procédé comprenant la détermination des niveaux de CSF3 et/ou CSF3R dans un échantillon de fluide conjointement avec IL-17A et/ou PlGF, dans lequel des niveaux élevés de CSF3 par rapport à un témoin indiquent une faible probabilité d'une grossesse réussie et des niveaux réduits de CSF3R par rapport à un témoin indiquent une faible probabilité de d'une grossesse réussie et des niveaux élevés d'IL-17A et/ou de PlGF par rapport à des témoins indiquent une faible probabilité de grossesse réussie et des niveaux normaux de CSF3 indiquant une probabilité élevée d'une issue de grossesse favorable et des niveaux normaux de CSF3R indiquant une probabilité élevée d'une issue de grossesse favorable et des niveaux normaux d'IL-17A et/ou de PlGF indiquant une probabilité élevée d'une issue de grossesse favorable, le tout par rapport à des témoins, dans lequel l'échantillon de fluide est sélectionné dans la liste constitué du lavage utérin, sang, plasma ou sérum, ascite, lymphe, exsudat tissulaire et urine.

2. Procédé selon la revendication 1, comprenant la détermination des niveaux de CSF3 et/ou de CSF3R dans un échantillon de fluide conjointement avec IL-17A et PlGF.

3. Procédé selon la revendication 1 ou la revendication 2, dans lequel un ou plusieurs des FCEV, IL-6, IL-8, CRP, sFlt-1 et sGP130 sont également dosés, dans lequel une élévation de l'un ou de plusieurs de ces biomarqueurs par rapport à un témoin indique une faible probabilité d'une issue de grossesse favorable.

4. Procédé selon la revendication 3, dans lequel un ou plusieurs parmi sFlt-1 et sGP130 sont dosés, dans lequel un niveau élevé du sFlt-1 et/ou du sGP130 par rapport à un témoin indique une faible probabilité d'une issue de grossesse favorable.

5. Procédé selon l'une quelconque des revendications 1 à 4, dans lequel le sujet féminin subit un cycle stimulé par des hormones.

6. Procédé selon l'une quelconque des revendications 1 à 4, dans lequel le sujet féminin subit un cycle d'ovulation naturel.

7. Procédé selon l'une quelconque des revendications 1 à 6, dans lequel l'échantillon de fluide est un échantillon de lavage utérin.

8. Procédé selon l'une quelconque des revendications 1 à 6, dans lequel l'échantillon de fluide est un échantillon de sang, de plasma ou de sérum.
